# EUROPEAN PATENT SPECIFICATION

(11) **EP 0 401 010 B1**
(45) Date of publication and mention of the grant of the patent: **21.08.1996**
(21) Application number: 90305920.2
(22) Date of filing: 31.05.1990
(51) Int. Cl.: C07D 311/68, C07D 405/04, C07D 491/04, A61K 31/35, C07D 405/14

(54) **Pyranyl cyanoguanidine derivatives**
Pyranyl-cyanoguanidin-Derivate
Dérivés pyranyliques de cyanoguanidines

(30) Priority: 31.05.1989 US 359236; 13.03.1990 US 493060
(43) Date of publication of application: 05.12.1990
(73) Proprietor: E.R. Squibb & Sons, Inc., Princeton, N.J. 08540-4000 (US)
(72) Inventor: Atwal, Karnail, Newtown, PA (US); Grover, Gary James, Stockton, NJ (US); Kim, Kyoung Soon, Lawrenceville, NJ (US)
(74) Representative: Thomas, Roger Tamlyn

(56) References cited:
- EP-A- 0 205 292
- EP-A- 0 214 818
- EP-A- 0 344 747
- EP-A- 0 350 805
- EP-A- 0 359 537
- EP-A- 0 389 861
- JOURNAL OF MEDICINAL CHEMISTRY, vol. 21, no. 8, August 1978, pp. 773-781, Washington, DC, US; H.J. PETERSEN et al.: "Synthesis and hypotensive activity of N-alkyl-N''-cyano-N'-pyridylguanidines"

## Description

### Field of the Invention

The present invention provides novel compounds having potassium channel activating activity which are therefore useful, for example, as cardiovascular agents.

### Summary of the Invention

In accordance with the present invention novel compounds having potassium channel activating activity which are useful, for example, as cardiovascular agents, are disclosed. These compounds have the general formula 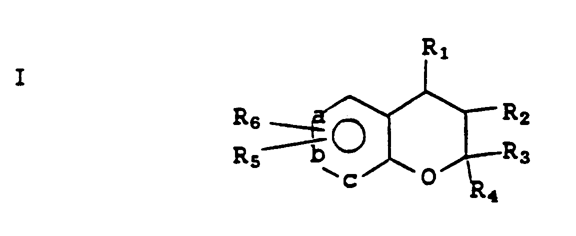 wherein a, b, and c are all carbons (=CH-) or one of a, b and c is nitrogen or =N(O)- and the others are =CH-;
R₁ is 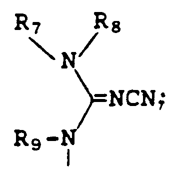 R₂ is hydrogen, hydroxy, 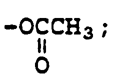 R₃ and R₄ are each independently hydrogen, alkyl or arylalkyl, or, R₃ and R₄ taken together with the carbon atom to which they are attached form a 5- to 7-membered carbocyclic ring;
R₅ is selected from H, alkyl, haloalkyl, alkenyl, alkynyl, cycloalkyl, arylalkyl, cycloalkylalkyl, -CN, -NO₂, -COR, -COOR, -CONHR, -CONR₂, -CF₃, S-alkyl, -SOalkyl, -SO₂alkyl, 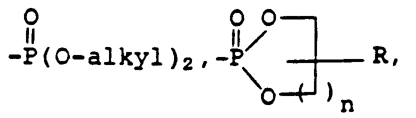 halogen, amino, substituted amino, O-alkyl, OCF₃, OCH₂CF₃, -OCOalkyl, -OCONRalkyl, -NRCOalkyl, NRCOOalkyl, and NRCONR₂ wherein R in each of the above groups can be hydrogen, alkyl, aryl, arylalkyl, cycloalkyl, or (cycloalkyl)alkyl;
R₆ is selected from H, alkyl, OH, O-alkyl, amino, substituted amino, CN, and NO₂;
R₇ is selected from aryl, heterocyclo, and (heterocyclo)alkyl;
R₈ is selected from hydrogen, alkyl, alkenyl, aryl, and arylalkyl;
R₉ is selected from hydrogen, alkyl, alkenyl, aryl, arylalkyl, cycloalkyl and cycloalkylalkyl; and
n is 1, 2 or 3.

### Detailed Description of the Present Invention

This invention in its broadest aspects relates to the cyanoguanidine compounds of formula I above, to compositions and the methods of using such compounds. The compounds of formula I are useful, for example, as cardiovascular agents. Preferred compounds are those with the 3S, 4R stereochemistry.

The term "alkyl" used in defining various symbols refers to straight or branched chain saturated hydrocarbon radicals having up to eight carbons, preferably from one to five carbons. Similarly, the terms "alkoxy" and "alkylthio" refer to such alkyl groups attached to an oxygen or sulfur.

The term "alkenyl" refers to straight or branched chain hydrocarbon radicals having from two to eight carbons and one double bond, preferably three to five carbons. The term "alkynyl" refers to straight or branched chain hydrocarbon radicals having from two to eight carbons and one triple bond, preferably three to five carbons.

The term "cycloalkyl" refers to saturated carbocyclic rings of 3 to 7 carbon atoms with cyclopropyl, cyclopentyl and cyclohexyl being most preferred.

The term "halo" or "halogen" refers to chloro, bromo and fluoro.

The term "halo substituted alkyl" refers to such alkyl groups described above in which one or more hydrogens have been replaced by chloro, bromo or fluoro groups such as trifluoromethyl, which is preferred,pentafluoroethyl, 2,2,2-trichloroethyl, chloromethyl, bromomethyl, etc.

The term "aryl" refers to phenyl, 1-naphthyl, 2-naphthyl or mono substituted phenyl, 1-naphthyl, or 2-naphthyl wherein said substituent is alkyl of 1 to 4 carbons, alkylthio of 1 to 4 carbons, alkoxy of 1 to 4 carbons, halo, nitro, cyano, hydroxy, amino, -NH-alkyl wherein alkyl is of 1 to 4 carbons, -N(alkyl)₂ wherein alkyl is of 1 to 4 carbons,
-CF₃, -OCHF₂, 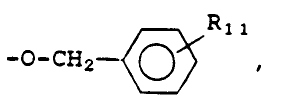 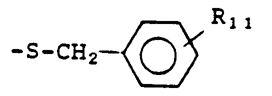 (wherein R₁₁ is hydrogen, alkyl of 1 to 4 carbons, alkoxy of 1 to 4 carbons, alkylthio of 1 to 4 carbons, halo, hydroxy or CF₃), -O-CH₂-cycloalkyl, or -S-CH₂- cycloalkyl, and di-substituted phenyl, 1-naphthyl, 2-naphthyl wherein said substituents are selected from methyl, methoxy, methylthio, halo, CF₃, nitro, amino, and OCHF₂.

Preferred aryl groups include unsubstituted phenyl and monosubstituted phenyl wherein the substituents are nitro, halo, -CF₃, alkyl, cyano or methoxy.

The term "heterocyclo" refers to fully saturated or unsaturated rings of 5 or 6 atoms containing one or two O and S atoms and/or one to four N atoms provided that the total number of hetero atoms in the ring is 4 or less. The hetero ring is attached by way of an available carbon atom. Preferred monocyclic hetero groups include 2- and 3-thienyl, 2- and 3-furyl, 2-, 3- and 4-pyridyl, and imidazolyl. The term hetero also includes bicyclic rings wherein a five or six membered ring containing O, S and N atoms as defined above is fused to a benzene ring and the bicyclic ring is attached by way of an available carbon atom. Preferred bicyclic hetero groups include 4, 5, 6, or 7-indolyl, 4, 5, 6, or 7-isoindolyl, 5, 6, 7 or 8-quinolinyl, 5, 6, 7 or 8-isoquinolinyl, 4, 5, 6, or 7-benzothiazolyl, 4, 5, 6 or 7-benzoxazolyl, 4, 5, 6 or 7-benzimidazolyl, 4, 5, 6 or 7-benzoxadiazolyl, and 4, 5, 6 or 7-benzofuranzanyl.

The term heterocyclo also includes such monocyclic and bicyclic rings wherein an available carbon atom is substituted with a lower alkyl of 1 to 4 carbons, lower alkylthio of 1 to 4 carbons, lower alkoxy of 1 to 4 carbons, halo, nitro, keto, cyano, hydroxy, amino, -NH-alkyl wherein alkyl is of 1 to 4 carbons, -N(alkyl)₂ wherein alkyl is of 1 to 4 carbons, CF₃, or OCHF₂ or such monocyclic and bicyclic rings wherein two or three available carbons have substituents selected from methyl, methoxy, methylthio, halo, CF₃, nitro, hydroxy, amino and OCHF₂.

The term "substituted amino" refers to a group of the formula -NZ₁Z₂ wherein Z₁ is hydrogen, alkyl, cycloalkyl, aryl, arylalkyl, cycloalkylalkyl and Z₂ is alkyl, cycloalkyl, aryl, arylalkyl, cycloalkylalkyl or Z₁ and Z₂ taken together with the nitrogen atom to which they are attached are 1-pyrrolidinyl, 1-piperidinyl, 1-azepinyl, 4-morpholinyl, 4-thiamorpholinyl, 1-piperazinyl, 4-alkyl-1-piperazinyl, 4-arylalkyl-1-piperazinyl, 4-diarylalkyl-1-piperazinyl, or 1-pyrrolidinyl, 1-piperidinyl, or 1-azepinyl substituted with alkyl, alkoxy, alkylthio, halo, trifluoromethyl or hydroxy.

The compounds of formula I can be prepared by treatment of a thiourea of the formula 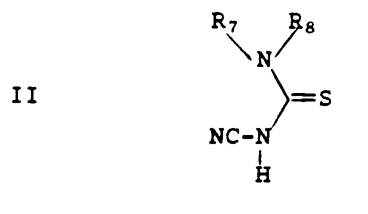 with an amine of the formula 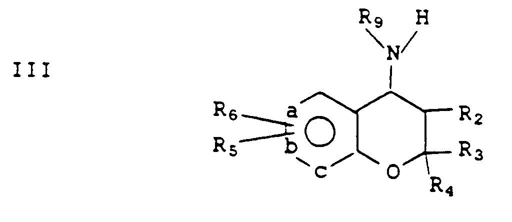 in the presence of a carbodiimide, preferably 1-(3-dimethylaminopropyl)-3-ethylcarbodiimide or dicyclohexylcarbodiimide and an acid source in an organic solvent, such as dimethylformamide, tetrahydrofuran, acetonitrile or dichloromethane.

The thiourea of formula II, wherein R₈ is hydrogen can be prepared by heating an isothiocyanate of the formula

R₇N=C=S IV

with either monosodium cyanamide or with cyanamide in the presence of an organic base, such as triethyl amine.

The other thioureas of formula II can be prepared by standard methods described in the literature, such as by C. R. Rasmussen, F. J. Villani, Jr., L. E. Weaner, B. E. Reynolds, A. R. Hood, L. R. Hecker, S. O. Nortey, A. Hanslin, M. J. Costanzo, E. T. Powell, A. J. Molinari, Synthesis, 1988, p. 456, and V. V. Mozolis and S. P. Locubaitite, Russian Chemical Reviews, 1973, 42, 587.

The aminoalcohol of formula III wherein R₂ is hydroxy can be prepared by methods described in the literature, such as by J. M. Evans, C. S. Fake, T. C. Hamilton, R. H. Poyser, E. A. Watts, J. Med. Chem. 1983, 26, 1582 and J. Med. Chem. 1986, 29, 2194; R. W. Lang, P. F. Wenk, Helvetica Chimica Acta, 1988, 71, 596; EP 0205292 A2 (1986), and WO 87/07607.

The amine of formula III, wherein R₂ is hydrogen, can be prepared from a ketone of the formula 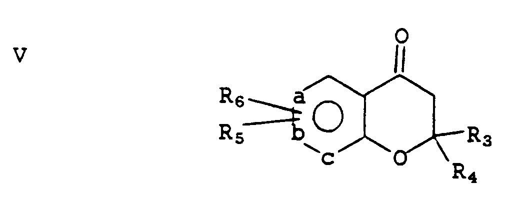 by standard methodology. The ketone of formula V can be obtained by literature procedures, such as disclosed by P. Sebok and T. Timar, Heterocycles, 1988, 27, 2595; P. Teixidor et al., Heterocycles, 1988, 27, 2459; A. Benerji and N. C. Goomer, Tetrahedron Letters, 1979, 3685; G. Ariamala and K. K. Subramanian, Tetrahedron Letters, Vol. 29, No. 28, p. 3487-3488 (1988).

The compounds of formula I can also be prepared by heating a thiourea of the formula 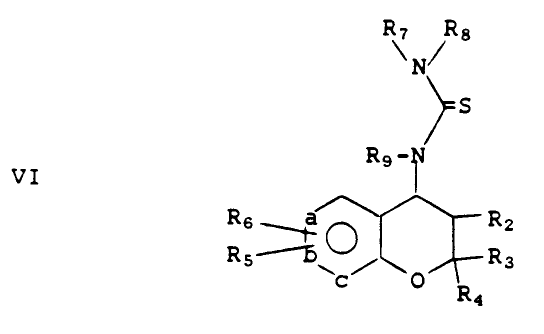 with monosodium cyanamide in the presence of a carbodiimide such as 1-(3-dimethylaminopropyl)-3-ethylcarbodiimide or dicyclohexylcarbodiimide in an organic solvent.

The compounds of formula VI can be prepared from the amino alcohol of formula III by standard methods (i.e., the Rasmussen and Mozolis references above).

The compounds of formula I can also be prepared by reacting a compound of the formula 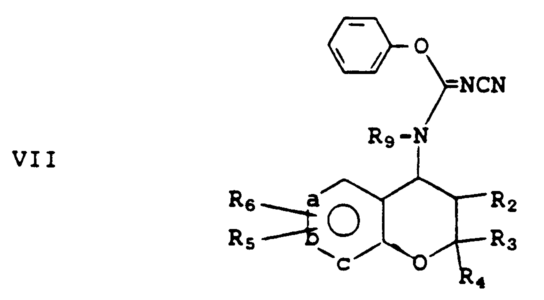 with an amine of the formula

R₇R₈NH VIII

in a polar solvent such as isopropanol. The compounds of formula VII are prepared by reacting an amine of formula III with diphenylcyanocarbonimidate.

The compounds of the present invention wherein R₂ is OCOalkyl can be prepared by acylation of the alcohol of formula I, wherein R₂ is OH, with an acid chloride of the formula 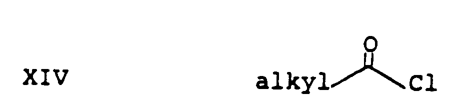 in the presence of a base catalyst, such as pyridine or triethylamine.

For the preparation of individual enantiomers of compounds of formula I (wherein R₂ =H, OH), compound III (R₂ = =H, OH) is converted to diastereomeric amides of the formula 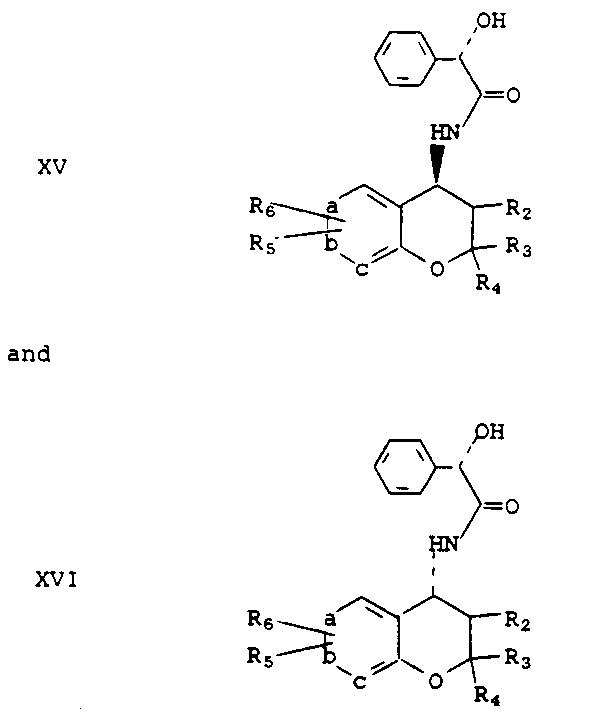 by treatment with chiral nonracemic mandelic acid in the presence of dicyclohexylcarbodiimide.

Compounds XV and XVI are separated by crystallization or chromatography. The enantiomer of mandelic acid that yields crystalline diastereomer with the desired 4R-stereochemistry of benzopyran (as shown in formula XV) is preferred in the resolution step.

Compounds XV and XVI are then hydrolyzed by heating in the presence of sulfuric acid in dioxane to give enantiomers of the formula

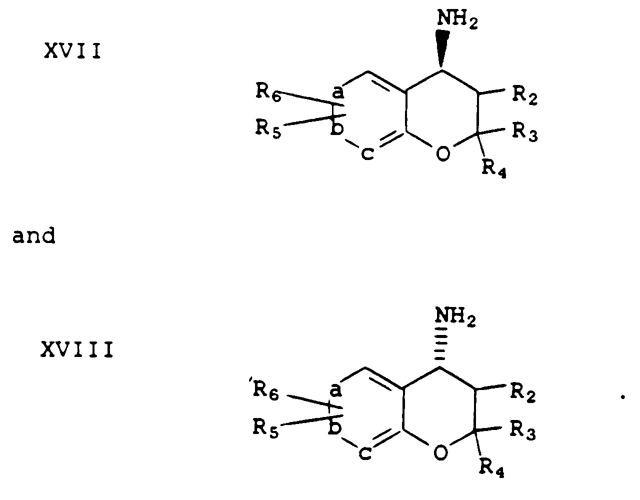

The enantiomers XVII and XVIII are then converted to chiral nonracemic compounds of formula I.

The compounds of the present invention can have asymmetric centers at carbons 2-4 of benzopyran ring. Also, any one of the R's can have an asymmetric carbon. Consequently, compounds of formula I can exist in diastereomeric forms or in mixtures thereof. The above described process can utilize racemates, enantiomers or diastereomers as starting materials. When diastereomeric products are prepared, they can be separated by conventional chromatographic or fractional crystallization methods.

The compounds of the present invention wherein R₉ and/or R₈ is hydrogen, can exist as a mixture of tautomers represented by the following structures. The tautomeric products are obtained in relative amounts that differ from compound to compound. All forms are included in the scope of formula I.

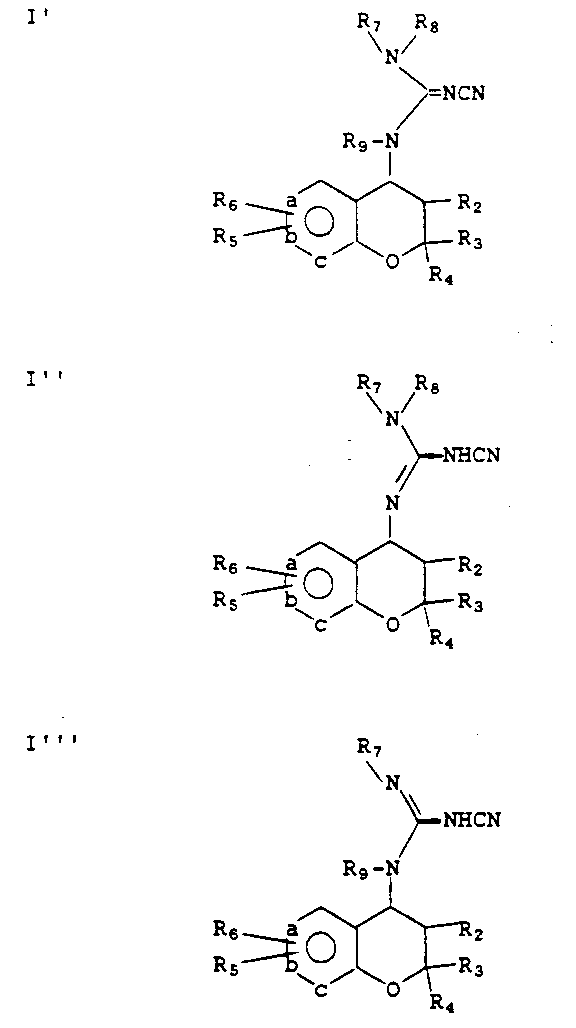

The compounds of formula I and the pharmaceutically acceptable salts act as potassium channel activators. Thus, compounds of the present invention are useful as anti-arrhythmic agents, antiischemic agents and in the treatment of hypertension.

In particular, it has been found that compounds of formula I can be used as antiischemic agents, i.e., for the treatment of ischemic conditions such as myocardial ischemia, cerebral ischemia, lower limb ischemia and the like. Especially preferred are those compounds where R₇ is aryl and R₃ and R₉ are each hydrogen. While any of the compounds of formula I may be used as antiischemic agents, these preferred antiischemic agents have been found to possess little or no vasodilator activity. This means that in the treatment of ischemic heart, these compounds are less likely to cause coronary steal, profound hypotension and coronary underperfusion.

Thus, for example, by the administration of a composition containing one (or a combination) of the compounds of this invention, ischemic conditions of a mammalian (e.g., human) host are reduced. A single dose, or preferably two to four divided daily doses, provided on a basis of about 0.001 to 100 mg per kilogram of body weight per day, preferably from about 0.1 to about 25 mg per kilogram per day, is appropriate to reduce ischemic conditions. The substance is preferably administered orally, but parenteral routes, such as the subcutaneous, intramuscular, or intravenous routes or any other convenient delivery system, such as inhalation or intranasal solutions or transdermal patches, can also be employed. The above doses are also suitable for the other cardiovascular (e.g., hypertension) and non-cardiovascular uses.

As a result of the potassium channel activating activity of compounds of this invention, these compounds are also useful in the treatment of cardiovascular disorders and any disorders associated with smooth muscle contraction. For example, compounds of the present invention are useful as therapy for congestive heart failure, therapy for peripheral vascular disorders (e.g. Raynaud's Disease), therapy for pulmonary hypertension, as anti-anginal agents, as anti-fibrillatory agents, as thrombolytic agents and in limiting myocardial infarction.

Compounds of the present invention are additionally expected to be useful in the treatment of central nervous system disorders (e.g., Parkinsonism, as anti-tremor agents, epilepsy), in therapy for renal failure, in therapy for urinary incontinence, as anti-diarrheal agents, in therapy for pre-eclampsia, dysmenorrhea and premature labor, as well as for the promotion of hair growth (e.g., in the treatment of male pattern baldness) and as anti-asthmatic agents.

The compounds of this invention can also be formulated in combination with a diuretic such as, chlorothiazide, hydrochlorothiazide, flumethiazide, hydroflumethiazide, bendroflumethiazide, methylchlothiazide, trichloromethiazide, polythiazide or benzthiazide as well as ethacrynic acid, tricrynafen, chlorthalidone, furosemide, musolimine, bumetanide, triamterene, amiloride and spironolactone and salts of such compounds, angiotensin converting enzyme inhibitors such as captopril, zofenopril, fosinopril, enalapril, ceranopril, cilazopril, delapril, pentopril, quinapril, ramipril, lisinopril, and salts of such compounds, thrombolytic agents such as tissue plasminogen activator (tPA), recombinant tPA, streptokinase, urokinase, prourokinase, and anisoylated plasminogen streptokinase activator complex (APSAC, Eminase, Beecham Laboratories), or calcium channel blocking agents such as nifedipine or diltiazem. Such combination products if formulated as a fixed dose employ the compounds of this invention within the dose range described above and the other pharmaceutically active agent within its approved dose range.

The compounds of formula I, and combinations thereof, can be formulated, as described above, in compositions such as tablets, capsules or elixirs for oral administration, in sterile solutions or suspensions for parenteral administration, and may also be administered via transdermal patch or nasal inhalation solutions. About 10 to 500 milligrams of a compound of formula I is compounded with physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Preferred compounds are those wherein
a is nitrogen or =CR₅-;
b and c are each =CH-;
R₂ is hydroxy or -OCOCH₃;
R₃ and R₄ are each alkyl;
R₅ is an electron withdrawing group;
R₆ is hydrogen, alkyl, O-alkyl, or amino;
R₇ is aryl;
R₈ is hydrogen;
R₉ is hydrogen or alkyl; and
n is 1 or 2.

Most preferred are those compounds wherein:
a is nitrogen or =CR₅-;
b and c are each =CH-;
R₂ is transhydroxy;
R₃ and R₄ are each methyl;
R₅ is -CN or -NO₂;
R₆ is hydrogen;
R₇ is phenyl or monosubstituted phenyl wherein the substituent is nitro, halo, -CF₃, alkyl, cyano or methoxy;
R₈ is hydrogen;
R₉ is hydrogen; and
n is 1.

The preferred compound of the present invention, which is preferably employed as an antiischemic agent, is

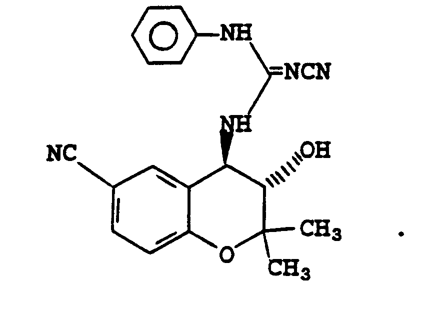

Specific embodiments of the present invention are described hereinafter in the following examples.

### Example 1

### (trans)-N''-Cyano-N-(6-cyano-3,4-dihydro-3-hydroxy-2,2-dimethyl-2H-1-benzopyran-4-yl)-N'-phenyl guanidine

### A. N-cyano-N'-phenylthiourea

To a suspension of monosodium cyanamide (6.4 g, 100 mmol) in absolute ethanol (170 mL), phenylisothiocyanate (12.5 mL, 104.5 mmol) was added slowly with stirring at room temperature. The reaction was allowed to stir at room temperature for 1 hour and then heated at 75°C for 4 hours. The reaction was cooled to room temperature and the colorless solid was filtered and washed with ethanol to give the title A compound (13.6 g), m.p. >250°C.

### B. (trans)-N''-Cyano-N-(6-cyano-3,4-dihydro-3-hydroxy-2,2-dimethyl-2H-1-benzopyran-4-yl)-N'-phenyl guanidine

To a solution of the title A compound (1.06 g, 5.96 mmol) and (*trans*)-4-amino-3,4-dihydro-3-hydroxy-2,2-dimethyl-2H-1-benzopyran-6-carbonitrile (prepared according to Evans et al., J. Med. Chem., 1983, 26, 1582 and J. Med. Chem., 1986, 29, 2194). (1.0 g, 4.59 mmol) in dimethylformamide (5 mL) under argon, 1-(3-dimethylaminopropyl)-2-ethylcarbodiimide hydrochloride (1.17 g, 5.96 mmol) was added at room temperature. The reaction mixture was stirred at room temperature for 2 hours and then partitioned between 1N HCl and ethyl acetate. The organic phase was separated and the aqueous phase was reextracted with ethyl acetate and the combined organic phase was washed with water, aqueous sodium bicarbonate and brine. After drying over anhydrous magnesium sulfate, the solvent was evaporated and the colorless residue was triturated with ether to yield the title compound (1.3 g), m.p. 247-249°C (with effervescence).

| Analysis calc'd for C₂₀H₁₉N₅O₂: | | | |
|---|---|---|---|
| | C, 66.46; | H, 5.30; | N, 19.38; |
| Found: | C, 66.09; | H, 5.30; | N, 19.35. |

### Example 2

### (trans)-N''-Cyano-N-(3,4-dihydro-3-hydroxy-2,2-dimethyl-2H-pyrano[3,2-c]pyridin-4-yl)-phenylguanidine

To a solution of the title A compound from Example 1 (2.2 g, 12.5 mmol) and (*trans*)-4-amino-3,4-dihydro-2,2-dimethyl-2H-pyrano[3,2-c]pyridin-3-ol (1.1 g, 5.7 mmol) (prepared according to EP 0 205 292 A2) in dimethylformamide (5 ml) under argon, 1-(3-dimethylaminopropyl)-2-ethylcarbodiimide hydrochloride (2.2 g, 10.8 mmol) was added at room temperature. The reaction mixture was stirred at room temperature for 2 hours and then partitioned between water (pH ∼ 11) and ethyl acetate. The organic phase was separated and the aqueous phase was reextracted with ethyl acetate and the combined organic phase was washed with water, aqueous sodium bicarbonate and brine. After drying over anhydrous magnesium sulfate, the solvent was evaporated and the residue was purified by flash chromatography on silica gel (acetone:dichloromethane/1:4) to yield a colorless solid (470 mg) which was crystallized from acetonitrile to provide the title compound, m.p. 233-236°C.

| Analysis calc'd for C₁₈H₁₉N₅O₂: | | | |
|---|---|---|---|
| | C, 64.08; | H, 5.67; | N, 20.76; |
| Found: | C, 63.88; | H, 5.48; | N, 20.76. |

### Example 3

### (3S-trans)-N''-Cyano-N-(6-cyano-3,4-dihydro-3-hydroxy-2,2-dimethyl-2H-1-benzopyran-4-yl)-N'-phenylguanidine

### A. [3R-[3α,4β(S*)]]-N-(6-Cyano-3,4-dihydro-3-hydroxy-2,2-dimethyl-2H-1-benzopyran-4-yl)-α-hydroxybenzeneacetamide

and

### [3S-[3α,4β(R*)]]-N-(6-Cyano-3,4-dihydro-3-hydroxy-2,2-dimethyl-2H-1-benzopyran-4-yl)-α-hydroxybenzeneacetamide

To a solution of (*trans*)-4-amino-3,4-dihydro-3-hydroxy-2,2-dimethyl-2H-1-benzopyran-6-carbonitrile (prepared according to Evans et al., J. Med. Chem., 1983, 26, 1582 and J. Med. Chem., 1986, 29, 2194) (10.0 g, 45.9 mmol), S-(+)-mandelic acid (6.98 g, 45.9 mmol), hydroxybenzotriazole hydrate (6.2 g, 45.9 mmol) in dimethylformamide (60 ml) at 0°C was added dicyclohexylcarbodiimide (9.5 g, 45.9 mmol). It was allowed to stir at room temperature for 20 hours and then cooled in an ice bath. The precipitated solid was filtered and the filtrate was concentrated *in vacuo*. The residue was dissolved in 5 percent methanol in chloroform and washed with 1 N sodium hydroxide, 1 N hydrochloric acid, brine and dried over anhydrous magnesium sulfate. After removing drying agent, the solvent was removed *in vacuo*. The residue was crystallized from ethanol to give [3R-[3α,4β(S*)]]-N-(6-cyano-3,4-dihydro-3-hydroxy-2,2-dimethyl-2H-1-benzopyran- 4-yl)-α-hydroxybenzeneacetamide (6.0 g) as a white solid, m.p. 238-240°C, [α_{D}]²⁵ = +94.6° (c = 1, MeOH): ¹H NMR (CDCl₃) δ 7.4 (m, 5 H), 7.26 (t, J = 1.0 HZ, 1 H), 6.97 (d, J = 9.0 HZ, 1 H), 6.83 (d, J = 9.0 HZ, 1 H), 5.16 (s, 1 H), 4.98 (t, J = 9.0 HZ, 1 H, 3.8 (d, J = 5.0 HZ, 1 H), 3.55 (dd, J = 4.0 & 5.0 HZ, 1 H), 1.45 (s, 3 H), 1.2 (s, 3 H).

| Analysis calc'd for C₂₀H₂₀N₂O₄: | | | |
|---|---|---|---|
| | C, 68.17; | H, 5.72; | N, 7.95; |
| Found: | C, 67.92; | H, 5.49; | N, 8.05. |

The residual material of the mother liquor was purified by flash chromatography on silica gel eluting with hexane-ethyl acetate mixture (3:7) and the residue was crystallized from dichloromethane-isopropyl ether to give [3S-[3α,4β(R*)]]-N-(6-cyano-3,4-dihydro-3-hydroxy-2,2-dimethyl-2H-1-benzopyran-4-yl)-α-hydroxybenzeneacetamide (6.0 g) as a white solid, m.p. 100-102°C (foaming); [α_{D}]²⁵ = -26.1° (c = 1, MeOH): ¹H NMR (DMSO-d₆) δ 8.45 (d, J = 8.0 Hz, 1 H), 7.5 (m, 4 H), 7.3 (m, 2 H), 7.0 (s, 1 H), 6.88 (d, J = 8.0 Hz, 1 H), 6.2 (s, 1 H), 5.57 (d, J = 5.0 Hz, 1 H), 5.0 (s, 1 H), 4.76 (t, J = 9.0 Hz, 1 H), 3.75 (dd, J = 5.0 & 5.0 Hz, 1 H), 1.40 (S, 3 H), 1.15 (s, 3 H).

| Analysis calc'd for C₂₀H₂₀N₂O₄·0.25 H₂O: | | | |
|---|---|---|---|
| | C, 67.30; | H, 5.78; | N, 7.84; |
| Found: | C, 67.54; | H, 5.95; | N, 7.44. |

### B. (3S-trans)-4-Amino-3,4-dihydro-3-hydroxy-2,2-dimethyl-2H-1-benzopyran-6-carbonitrile

To a solution of [3S-[3α,4β(R*)]]-N-(6-cyano-3,4-dihydro-3-hydroxy-2,2-dimethyl-2H-1-benzopyran-4-yl)-α-hydroxybenzeneacetamide, title A compound (2.8 g, 7.9 mmol) in dioxane (30 ml) was added a solution of sulfuric acid (2.5 g) in water (12 ml) at room temperature and the reaction mixture was heated at reflux temperature for 24 hours. It was concentrated *in vacuo* and the residue was dissolved in ethyl acetate (200 ml). The organic layer was washed with 1 N sodium hydroxide (50 ml) followed by water (50 ml) and dried over anhydrous magnesium sulfate and concentrated *in vacuo* to give the title B compound (1.6 g) as an oil: ¹H NMR (CDCl₃) δ 7.74 (s, 1 H), 7.42 (dd, J = 2.0 & 6.0 Hz, 1 H), 6.82 (d, J = 8.0 Hz, 1 H), 3.65 (d, J = 10.0 Hz, 1 H), 3.36 (d, J = 10.0 Hz, 1 H), 1.53 (s, 3 H), 1.23 (s, 3 H).

### C. (3S-trans)-N˝-cyano-N-(6-cyano-3,4-dihydro-3-hydroxy-2,2-dimethyl-2H-1-benzopyran-4-yl)-N′-phenylguanidine

To a solution of N-cyano-N′-phenylthiourea (1.7 g, 9.5 mmol) and the title B compound (1.6 g, 7.3 mmol) in dimethylformamide (7 mL), under argon was added 1-(3-dimethylaminopropyl)-2-ethylcarbodiimide hydrochloride (1.8 g, 9.5 mmol) at room temperature. The reaction mixture was stirred at room temperature for 2 hours and then partitioned between 1N hydrochloric acid and ethyl acetate. The organic phase was separated and the aqueous phase was reextracted with ethyl acetate. The combined extracts were washed with water, aqueous sodium bicarbonate and brine. After drying over anhydrous magnesium sulfate, the solvent was evaporated and the crude product was purified by flash chromatography on silica gel eluting with ethyl acetate/hexanes (7:3) to give a cololess solid (0.7 g). The solid was triturated with ether to yield the title compound (0.35 g) , m.p. 214-216°C; [α_{D}]²⁵ = -34.8° (c = 0.417, MeOH): ¹H NMR (DMSO-d₆) d 9.28 (s, 1 H), 7.58 (d, J = 8.0 Hz, 3 H), 7.35 (m, 4 H), 7.15 (m, 1 H), 6.90 (d, J = 8.2 Hz, 1 H), 5.92 (br s, 1 H), 4.92 (t, J = 9.0 Hz, 1 H), 3.72 (br d, J = 5.9 Hz, 1 H), 1.41, 1.18 (s, 3 H each); ¹³C NMR (DMSO-d₆) 159.2, 156.3, 137.5, 132.6, 132.5, 129.0, 124.8, 124.7, 123.6, 119.0, 117.8, 117.0, 102.6, 80.4, 70.9, 51.9, 26.6, 18.6; IR(KBr) 2226, 2179, 1609, 1582, 1491, 1267 cm⁻¹.

| Analysis calc'd for C₂₀H₁₉N₅O₂·0.37 H₂O: | | | |
|---|---|---|---|
| | C, 65.26; | H, 5.40; | N, 19.02; |
| Found: | C, 65.62; | H, 5.36; | N, 18.57. |

### Example 4

### (3R-trans-N˝-Cyano-N-(6-cyano-3,4-dihydro-3-hydroxy-2,2-dimethyl-2H-1-benzopyran-4-yl)-N′-phenylguanidine

### A. (3R-trans)-4-amino-3,4-dihydro-3-hydroxy-2,2-dimethyl-2H-1-benzopyran-6-carbonitrile

To a solution of [3R-[3α,4β(S*)]]-N-(6-cyano-3,4-dihydro-3-hydroxy-2,2-dimethyl-2H-1-benzopyran-4-yl)-α-hydroxybenzeneacetamide, compound of Example 3, part A (2.8 g, 7.9 mmol) in dioxane (30 ml) were added concentrated sulfuric acid (2.5 g) and water (12 ml} at room temperature and the reaction mixture was heated at reflux temperature for 24 hours. It was concentrated *in vacuo* and the residue was combined with another batch of the same material and dissolved in ethyl acetate (400 ml). The resulting solution was washed with 1N sodium hydroxide (50 ml) followed by water (50 ml) and dried over anhydrous magnesium sulfate and concentrated *in vacuo* to give the title A compound (3.7 g) as an oil: ¹H NMR (CDCl₃) δ 7.74 (s, 1 H), 7.42 (dd, J = 2.0 & 6.0 Hz, 1 H), 6.82 (d, J = 8.0 Hz, 1 H), 3.65 (d, J = 10.0 Hz, 1 H), 3.36 (d, J = 10.0 Hz, 1 H), 1.53 (s, 3 H), 1.23 (s, 3 H).

### B. (3R-trans)-N˝-Cyano-N-(6-cyano-3,4-dihydro-3-hydroxy-2,2-dimethyl-2H-1-benzopyran-4-yl)-N′-phenylguanidine

To a solution of N-cyano-N′-phenylthiourea (3.9 g, 21.9 mmol) and the title A compound (3.68 g, 16.9 mmol) in dimethylformamide (20 mL) under argon, 1-(3-dimethylaminopropyl)-2-ethylcarbodiimide hydrochloride (4.2 g, 21.9 mmol) was added at room temperature. The reaction mixture was stirred at room temperature for 2 hours and then partitioned between 1N hydrochloric acid and ethyl acetate. The organic phase was separated and the aqueous phase was reextracted with ethyl acetate. The combined extracts were washed with water, aqueous sodium bicarbonate and brine. After drying over anhydrous magnesium sulfate, the solvent was evaporated and the crude product was triturated with ethyl acetate-ether to give a colorless solid (3.5 g). The crude product was purified by flash chromatography on silica gel eluting with ethyl acetate/hexanes (7:3) and the solid, obtained after evaporation of the solvent, was triturated with ether to give the title compound (1.8 g) as a colorless solid, m.p. 215-217°C, [α_{D}]²⁵ = +34.8° (c = 0.417, MeOH): ¹H NMR (CDCl₃/DMSO-d₆) δ 8.8 (s, 1 H), 7.6 (s, 1 H), 7.44 (d, J = 8.0 Hz, 1 H), 7.35 (d, J = 5.0 Hz, 4 H), 7.22 (m, 1 H), 6.85 (d, J = 8.8 Hz, 1 H), 6.7 (br s, 1 H), 5.0 (t, J = 9.0 Hz, 1 H), 3.72 (br d, J = 5.3 Hz, 1 H), 1.48, 1.18 (s, 3 H each); ¹³C NMR (CDCl₃/DMSO-d₆) 159.6, 156.5, 136.6, 132.5, 129.2, 125.7, 124.1, 123.7, 118.9, 118.1, 117.2, 103.4, 80.3, 72.8, 52.4, 26.4, 18.6; IR (KBr) 2226, 2179, 1609, 1582, 1491, 1267 cm⁻¹.

| Analysis calc'd for C₂₀H₁₉N₅O₂·0.45 H₂O: | | | |
|---|---|---|---|
| | C, 65.01; | H, 5.42; | N, 18.95; |
| Found: | C, 65.18; | H, 5.47; | N, 18.51. |

Example 5 is an alternate procedure to Example 3 and the procedure of this Example 5 is preferred. Additionally, the 3S, 4R enantiomer of Example 5 is the preferred compound of the present invention.

### Example 5

### (3S-trans)-N˝-Cyano-N-(6-cyano-3,4-dihydro-3-hydroxy-2,2-dimethyl-2H-1-benzopyran-4-yl)-N′-phenylguanidine

### A. [3S-[3α,4β(S*)]]-N-(6-Cyano-3,4-dihydro-3-hydroxy-2,2-dimethyl-2H-1-benzopyran-4-yl)-α-hydroxybenzeneacetamide

and

### [3R-[3α,4β(R*)]]-N-(6-Cyano-3,4-dihydro-3-hydroxy-2,2-dimethyl-2H-1-benzopyran-4-yl)-α-hydroxybenzeneacetamide

To a solution of (*trans*)-4-amino-3,4-dihydro-3-hydroxy-2,2-dimethyl-2H-1-benzopyran-6-carbonitrile (prepared according to Evans et al., J. Med. Chem., 1983, 26, 1582 and J. Med. Chem., 1986, 29, 2194) (1.64 g, 7.5 mmol), R(-)-mandelic acid (1.14 g, 7.5 mmol), hydroxybenzotriazole hydrate (1.0 g, 7.5 mmol) in dimethylformamide (15 ml) at 0°C was added dicyclohexylcarbodiimide (1.55 g, 7.5 mmol) at room temperature. The reaction mixture was allowed to stir at room temperature for 20 hours and then cooled in an ice bath. The solid was removed by filtration and the filtrate was concentrated *in vacuo*. The residue was dissolved in 5% methanol in chloroform and washed with 1 N sodium hydroxide, 1 N hydrochloric acid, brine followed by drying over anhydrous magnesium sulfate. After removing drying agent the solvent was removed *in vacuo*. The residue was crystallized from ethanol to give [3S-[3α,4β(S*)]]-N-(6-cyano-3,4-dihydro-3-hydroxy-2,2-dimethyl-2H-1-benzopyran-4-yl)-α-hydroxybenzeneacetamide (0.85 g) as a white solid, m.p. 235-237°C: [α_{D}]²⁵ = -94.9° (c = 1, MeOH); ¹H NMR (DMSO-d₆) δ 8.45 (d, J = 8.0 Hz, 1 H), 7.5 (m, 4 H), 7.3 (m, 2 H), 7.0 (s, 1 H), 6.88 (d, J = 8.0 Hz, 1 H), 6.2 (s, 1 H), 5.57 (d, J = 5.0 Hz, 1 H), 5.0 (s, 1 H), 4.76 (t, J = 9.0 Hz, 1 H), 3.75 (dd, J = 5.0 & 5.0 Hz, 1 H), 1.40 (s, 3 H), 1.15 (s, 3 H).

| Analysis calc'd for C₂₀H₂₀N₂O₄: | | | |
|---|---|---|---|
| | C, 68.17; | H, 5.72; | N, 7.95; |
| Found: | C, 68.00; | H, 5.52; | N, 7.95. |

The residual material recovered from the mother liquor was purified by flash chromatography on silica gel eluting with hexane-ethyl acetate (3:7) and the product was crystallized from dichloromethane-isopropyl ether to give [3R-[3α,4β(R*)]]-N-(6-Cyano-3,4-dihydro-3-hydroxy-2,2-dimethyl-2H-1-benzopyran-4-yl)-α-hydroxybenzeneacetamide as a white solid, m.p. 100-102°C (foaming): [α_{D}]²⁵ = +25.6° (c = 1, MeOH): ¹H NMR (CDCl₃) δ 7.4 (m, 5 H), 7.26 (t, J = 1.0 Hz, 1 H), 6.97 (d, J = 9.0 Hz, 1 H), 6.83 (d, J = 9.0 Hz, 1 H), 5.16 (s, 1 H), 4.98 (t, J = 9.0 Hz, 1 H), 3.8 (d, J = 5.0 Hz, 1 H), 3.55 (dd, J = 4.0 & 5.0 Hz, 1 H), 1.45 (s, 3 H), 1.2 (s, 3 H).

| Analysis calc'd for C₂₀H₂₀N₂O₄·0.25 H₂O: | | | |
|---|---|---|---|
| | C, 67.30; | H, 5.78; | N, 7.84; |
| Found: | C, 67.17; | H, 5.87; | N, 7.44. |

### B. (3S-trans)-4-Amino-3,4-dihydro-3-hydroxy-2,2-dimethyl-2H-1-benzopyran-6-carbonitrile

To a solution of [3S-[3α,4β(S*)]]-N-(6-cyano-3,4-dihydro-3-hydroxy-2,2-dimethyl-2H-1-benzopyran-4-yl)-α-hydroxybenzeneacetamide, title A compound (6.09 g, 17.0 mmol) in dioxane (60 ml) was added a solution of sulfuric acid (6.0 g) in water (30 ml) at room temperature and the reaction mixture was heated at reflux temperature for 24 hours. It was then concentrated *in vacuo* and the residue was dissolved in ethyl acetate. The organic layer was washed with 1N sodium hydroxide followed by water and dried over anhydrous magnesium sulfate. The solvent was evaporated to give the title B compound as an oil: ¹H NMR (CDCl₃) δ 7.74 (s, 1 H), 7.42 (dd, J = 2.0 & 6.0 Hz, 1 H), 6.82 (d, J = 8.0 Hz, 1 H), 3.65 (d, J = 10.0 Hz, 1 H), 3.36 (d, J = 10.0 Hz, 1 H), 1.53 (s, 3 H), 1.23 (s, 3 H).

### C. (3S-trans)-N˝-Cyano-N-(6-cyano-3,4-dihydro-3-hydroxy-2,2-dimethyl-2H-1-benzopyran-4-yl)-N′-phenylguanidine

To a solution of N-cyano-N′-phenylthiourea (2.11 g, 11.9 mmol) and (3S-trans)-4-amino-3,4-dihydro-3-hydroxy-2,2-dimethyl-2H-1-benzopyran-6-carbonitrile (2.0 g, 9.1 mmol), title B compound, in dimethylformamide (20 mL) under argon was added 1-(3-dimethylaminopropyl)-2-ethylcarbodiimide hydrochloride (2.23 g, 11.9 mmol) at room temperature. The reaction mixture was stirred at room temperature for 2 hours and then partitioned between 1N hydrochloric acid and ethyl acetate. The organic phase was separated and the aqueous phase was reextracted with ethyl acetate. The combined organic extracts were washed with water, sodium bicarbonate and brine. After drying over anhydrous magnesium sulfate, the solvent was evaporated and the crude product was purified by flash chromatography on silica gel eluting with ethyl acetate/hexanes (7:3) to give a colorless solid which was triturated with ether to yield the title compound (0.35 g), m.p. 215-216°C: [α]_{D}²⁵ = -33.5° (c = 1, MeOH); ¹H NMR (DMSO-d₆) δ 9.28 (s, 1 H), 7.58 (d, J = 8.0 Hz, 3 H), 7.35 (m, 4 H), 7.15 (m, 1 H), 6.90 (d, J = 8.2 Hz, 1 H), 5.92 (br s, 1 H), 4.92 (t, J = 9.0 Hz, 1 H), 3.72 (br d, J = 5.9 Hz, 1 H), 1.41, 1.18 (s, 3 H each); ¹³C NMR (DMSO-d₆) 159.2, 156.3, 137.5, 132.6, 132.5, 129.0 124.8, 124.7, 123.6, 119.0, 117.8, 117.0, 102.6, 80.4, 70.9, 51.9, 26.6, 18.6; IR (KBr) 2226, 2179, 1609, 1582, 1491, 1267 cm⁻¹.

| Analysis calc'd for C₂₀H₁₉N₅O₂·0.24 H₂O: | | | |
|---|---|---|---|
| | C, 65.26; | H, 5.40; | N, 19.02; |
| Found: | C, 65.62; | H, 5.36; | N, 18.57. |

HPLC: 99.5% by Chiracel OD column/hexanes (80%), isopropanol (20%), formic acid (0.1%).

### Example 6

### (trans)-N''-Cyano-N-(6-cyano-3,4-dihydro-3-hydroxy-2,2-dimethyl-2H-1-benzopyran-4-yl)-N'-(4-pyridinylmethyl)guanidine

### A. (trans)-4-[[(Cyanoimino)phenoxymethyl]amino]-3,4-dihydro-3-hydroxy-2,2-dimethyl-2H-1-benzopyran-6-carbonitrile

To a solution of (*trans*)-4-amino-3,4-dihydro-3-hydroxy-2,2-dimethyl-2H-1-benzopyran-6-carbonitrile (prepared according to Evans et al., J. Med. Chem., 1983, 26, 1582 and J. Med. Chem., 1986, 29, 2194) (5.0 g, 23 mmol) in isopropanol (50 mL), diphenylcyanocarbonimidate (5.5 g, 25 mmol) was added at room temperature and the reaction mixture was allowed to stir at room temperature for 16 hours. Most of the isopropanol was evaporated and the residue was dissolved in ethyl acetate. The resulting solution was washed successively with 10% citric acid, 1N sodium hydroxide solution and brine. It was dried over anhydrous magnesium sulfate, concentrated and the residue was crystallized from chloroform-isopropyl ether to yield the title A compound (4.2 g) as a colorless solid, m.p. 186-188°C.

| Analysis calc'd for C₂₀H₁₈N₄O₃·0.6H₂O: | | | |
|---|---|---|---|
| | C, 64.37; | H, 5.18; | N, 15.02; |
| Found: | C, 64.64; | H, 4.86; | N, 14.75. |

### B. (trans)-N''-Cyano-N-(6-cyano-3,4-dihydro-3-hydroxy-2,2-dimethyl-2H-1-benzopyran-4-yl)-N'-(4-pyridinylmethyl)guanidine

A suspension of (*trans*)-4[[(cyanoimino)-phenoxymethyl]amino]-3,4-dihydroxy-2,2-dimethyl-2H-1-benzopyran-6-carbonitrile (1.0 g, 2.76 mmol), compound of part A, in isopropanol (5 ml) was treated at room temperature with 4-(amino-methyl)pyridine (1.0 ml). The reaction mixture was allowed to stir at room temperature for 4 hours and then heated at reflux temperature for 16 hours. The reaction mixture was cooled to ambient temperature and the precipitated solid was filtered off. The product was recrystallized from ethyl acetate to give the title compound (0.76 g) as a colorless solid, m.p. 156-158°C: ¹H NMR (DMSO-d₆) δ 8.53 (d, J = 6.0 Hz, 2 H), 7.9 (m, 1 H), 7.59 (dd, J = 3.0 & 6.0 Hz, 1 H), 7.44 (s, 2 H), 7.31 (d, J = 6.0 Hz, 2 H), 6.91 (d, J = 9.0 Hz, 1 H), 5.9 (s, 1 H), 4.87 (m, 1 H), 4.48 (t, J = 2.0 & 6.0 Hz, 2 H), 3.7 (m, 1 H), 1.99 (s, 3 H), 1.18 (s, 3 H); ¹³C NMR (DMSO-d₆) 160.4, 156.2, 149.4, 132.6, 132.3, 124.7, 121.7, 117.8, 117.4, 102.6, 80.4, 71.0, 51.5, 43.4, 26.5, 18.6; IR (KBr) 1125.2, 1490.2, 1524.1, 1577.8, 1611.3, 2170.4, 2224.9, 3429.7 cm⁻¹.

| Analysis calc'd for C₂₀H₂₀N₆O₂·0.2 H₂O: | | | |
|---|---|---|---|
| | C, 63.22; | H, 5.41; | N, 22.12; |
| Found: | C, 63.42; | H, 5.17; | N, 21.92. |

### Example 7

### (trans)-N''-Cyano-N-(6-cyano-3,4-dihydro-3-hydroxy-2,2-dimethyl-2H-1-benzopyran-4-yl)-N'-(3-pyridinylmethyl)guanidine

A suspension of (*trans*)-4-[[(cyanoimino)-phenoxymethyl]amino]-3,4-dihydroxy-2,2-dimethyl-2H-1-benzopyran-6-carbonitrile (1.0 g, 2.76 mmol), compound of Example 6, part A, in isopropanol (5 ml) was treated at room temperature with 3-(aminomethyl)pyridine (1.0 ml). The reaction mixture was allowed to stir at room temperature for 4 hours and then heated under reflux for 16 hours. The reaction mixture was concentrated *in vacuo* and the resulting solid was crystallized from ethyl acetate to give the title compound (0.72 g) as a colorless solid, m.p. 226-228°C: ¹H NMR (DMSO-d₆) δ 8.55 (s, 1 H), 8.49 (d, J = 2.0 Hz, 2 H), 7.85 (m, 1 H), 7.75 (d, J = 8.0 Hz, 1 H), 7.59 (d, J = 8.0 Hz, 1 H), 7.40 (m, 3 H), 6.91 (d, J = 8.0 Hz, 1 H), 5.85 (s, 1 H), 4.82 (m, 1 H), 4.48 (m, 2 H), 3.74 (m, 1 H), 1.40 (s, 3 H), 1.17 (s, 3 H); ¹³C NMR 160.43, 156.2, 148.6, 148.2, 134.6, 134.2, 132.7, 132.2, 124.8, 123.4, 118.9, 117.9, 117.5, 102.6, 80.4, 71.0, 51.5, 42.1, 26.6, 18.7; IR (KBr) 1125.2, 1490.1, 1524.1, 1577.8, 1611.3, 2170.4, 2224.9, 3429.7 cm⁻¹.

| Analysis calc'd for C₂₀H₂₀N₆O₂·0.17 H₂O: | | | |
|---|---|---|---|
| | C, 63.22; | H, 5.41; | N, 22.12; |
| Found: | C, 63.08; | H, 5.32; | N, 22.38. |

### Example 8

### (trans)-N˝-Cyano-N-(6-ethynyl-3,4-dihydro-3-hydroxy-2,2-dimethyl-2H-1-benzopyran-4-yl)-N′-phenylguanidine

### A. 1-((1,1-Dimethyl-2-propynyl)oxy)-4-iodobenzene

A solution of 3-chloro-3-methyl-1-butyne (10.0 g, 97.9 mmol), 4-iodophenol (15.0 g, 68.4 mmol), sodium hydroxide (3.90 g, 97.5 mmol) and tetrabutylammonium hydrogen sulfate (9.33 g, 27.5 mmol) in methylene chloride (50 mL) and water (50 mL) was stirred for 19 days at room temperature. After separating the two layers, the organic layer was washed with 1 N sodium hydroxide followed by water, dried over magnesium sulfate and concentrated *in vacuo*. The residue was dissolved in ethyl acetate and washed successively with 1 N hydrochloric acid, 1 N sodium hydroxide, water, brine and dried over anhydrous magnesium sulfate. After removing drying agent, the solvent was removed *in vacuo*. The crude product was purified by flash chromatography on silica gel eluting with toluene/hexane (1:10) to give the title compound as an oil (5.78 g, 20.2 mmol) in 30% yield: ¹H NMR (CDCl₃) δ 7.56 (d, J = 8.7 Hz, 2H), 6.98 (d, J = 8.8 Hz, 2H), 2.56 (s, 1H), 1.63 (s, 6H); ¹³C NMR (CDCl₃) δ 155.4, 137.8, 123.5, 86.0, 85.6, 74.3, 72.6, 29.5.

### B. 2,2-Dimethyl-6-iodo-2H-1-benzopyran

The title A compound (3.91 g, 13.7 mmol) was heated in an oil bath at 170° for 2 hours. After cooling, the crude product was purified by flash chromatography on silica gel eluting with toluene/hexane (1:20) to give the title compound as an oil (3.26 g, 11.4 mmol) in 83% yield: ¹H NMR (CDCl₃) δ 7.34 (dd, J₁ = 1.8, J₂ = 2.4 Hz, 1H), 7.24 (d, J = 0.9 Hz, 1H), 6.52 (d, J = 8.8 Hz, 1H), 6.21 (d, J = 10.0 Hz, 1H), 5.58 (d, J = 10.0 Hz, 1H), 1.40 (s, 6H); ¹³C NMR (CDCl₃) δ 152.8, 137.5, 134.7, 131.6, 123.6, 121.1, 118.6, 82.4, 76.4, 27.9.

### C. 2,2-Dimethyl-6-(trimethylsilyl)ethynyl)-2H-1-benzopyran

A solution of the title B compound (1.32 g, 4.61 mmol), trimethyl((trimethylstannyl)ethynyl)-silane (1.60 g, 5.69 mmol), lithium chloride (0.62 g, 14.6 mmol) and tetrakis(triphenylphosphine) palladium (0.69 g, 0.60 mmol) in dioxane (16.5 mL) was stirred under argon for 5 hours in an oil bath at 65°. The reaction mixture was cooled to room temperature and concentrated *in vacuo* to give a residue that was combined with the material prepared in a similar manner on a 2.42 mmol scale. The combined material was rinsed with toluene/hexane (1:10) and the filtrate was concentrated *in vacuo*. The crude product was purified by flash chromatography on silica gel eluting with toluene/hexane (1:10) to give the title C cmpound as an oil (1.82 g, 7.00 mmol) in 100% yield: ¹H NMR (CDCl₃) δ 6.98 (dd, J₁ = 2.3, J₂ = 8.2 Hz, 1H), 6.87 (d, J = 1.8 Hz, 1H), 6.44 (d, J = 8.2 Hz, 1H), 6.02 (d, J = 9.4 Hz, 1H), 5.38 (d, J = 10.0 Hz, 1H), 1.20 (s, 6H), 0.00 (s, 9H); ¹³C NMR (CDCl₃) δ 153.4, 133.0,131.2, 130.0,121.6, 121.0, 116.3, 115.2, 105.2, 92.1, 76.7, 28.1, 0.1.

| Analysis calc'd for C₁₆H₂₀OSi: | | |
|---|---|---|
| | C, 74.94; | H, 7.86; |
| Found: | C, 75.19; | H, 7.61. |

### D. (cis)-1a,7b-Dihydro-2,2-dimethyl-6-((trimethylsilyl)ethynyl)-2H-oxireno(c)-(1)-benzopyran

To a solution of the title C compound (1.37 g, 5.34 mmol) and sodium bicarbonate (2.33 g, 27.7 mmol) in methylene chloride (27 mL) and water (27 mL) at 0° was added 3-chloroperoxybenzoic acid (1.51 g of 80-85% purity, 7.01 mmol). After a few minutes of stirring, the ice bath was removed and the reaction mixture was stirred at room temperature for 9 hours. After adding methylene chloride to the reaction mixture, the organic layer was separated and washed with water followed by brine, dried over magnesium sulfate and concentrated *in vacuo*. The crude product was purified by flash chromatography on silica gel eluting with hexane/ethyl acetate (10:1) to give recovered starting material (0.47 g) and the title copound as an oil (0.53 g, 1.95 mmol) in 36% yield: ¹H NMR (CDCl₃) δ 7.24 (d, J = 1.8 Hz, 1H), 7.11 (dd, J₁ = 1.78, J₂ = 2.3 Hz, 1H), 6.49 (d, J = 8.2 Hz, 1H), 3.62 (d, J = 4.1 Hz, 1H), 3.25 (d, J = 4.1 Hz, 1H), 1.34 (s, 3H), 1.00 (s, 3H), 0.00 (s, 9H); ¹³C NMR (CDCl₃) δ 152.9, 134.1, 133.4, 120.0, 118.1, 103.6, 92.9, 73.6, 62.5, 50.5, 25.6, 22.7, 0.00.

### E. (trans)-4-Amino-6-ethynyl-3,4-dihydro-2H-1-benzopyran-3-ol

A solution of the title D compound (0.53 g, 1.95 mmol) in ethanol (15 mL) and concentrated aqueous ammonium hydroxide (30 mL) was stirred at room temperature for 4 days. The solvent was removed *in vacuo* and the residue was purified by flash chromatography on silica gel eluting with hexane/ethyl acetate/methanol (5:5:1) to give a partially purified product. This material was triturated with diethyl ether to give the title compound as a white solid (0.42 g, 1.93 mmol) in 99% yield, m.p. 132-134°C: ¹H NMR (CDCl₃) δ 7.62 (s, 1H), 7.38 (dd, J₁ = 1.2, J₂ = 1.8 Hz, 1H), 6.82 (d, J = 8.2, Hz, 1H), 3.73 (d, J = 10.0 Hz, 1H), 3.45 (d, J = 9.4 Hz, 1 H), 3.10 (s, 1H), 2.56 (br s, 3H), 1.59 (s, 3H), 1.30 (s, 3H); ¹³C NMR (CDCl₃) δ 153.0, 132.6, 131.0, 125.7, 117.2, 114.0, 83.6 78.6, 75.9, 75.8, 51.1, 26.9, 18.7.

| Analysis calc'd for C₁₃H₁₅NO₂·0.06 H₂O: | | | |
|---|---|---|---|
| | C, 71.52; | H, 6.98; | N, 6.42; |
| Found: | C, 71.47; | H, 6.95; | N, 6.47. |

### F. (trans)-N˝-Cyano-N-(6-ethynyl-3,4-dihydro-3-hydroxy-2,2-dimethyl-2H-1-benzopyran-4-yl)-N′-phenylguanidine

To a solution of the title E compound (0.150 g, 0.69 mmol) and N-cyano-N′-phenylthiourea (0.180 g, 1.0 mmol) in dimethylformamide (5 mL) was added 1-(3-dimethylaminopropyl)-2-ethylcarbodiimide hydrochloride (0.200 g, 1.0 mmol) at room temperature. The reaction mixture was stirred overnight at room temperature and the solvent was removed *in vacuo*. The residue was partitioned between water and ethyl acetate. The organic layer was separated and dried over sodium sulfate. The solvent was removed *in vacuo* and the residue was purified by flash chromatography on silica gel eluting with hexane/ethyl acetate/ethanol (30:10:5) to give a partially pure product. This material was triturated with diethyl ether to give the title compound (0.12 g, 0.34 mmol) in 49% yield, m.p. 220-222°C (dec); ¹H NMR (CDCl₃) δ 7.20-7.40 (m, 7H), 6.70 (d, J = 8.2 Hz, 1H), 5.00 (d, J = 10.0 Hz, 1H), 3.67 (d, J = 9.4 Hz, 1H), 3.34 (s, 1H), 1.44 (s, 3H), 1.24 (s, 3H); ¹³C NMR (CDCl₃) δ 161.6, 154.6, 138.2, 133.7, 132.8 130.5, 127.2, 125.7, 123.9, 118.9, 118.3, 116.0, 84.3, 80.5, 77.2, 74.6, 54.0, 27.1, 18.6.

| Analysis calc'd for C₂₁H₂₀N₄O₂·0.32 H₂O: | | | |
|---|---|---|---|
| | C, 68.89; | H, 5.68; | N, 15.31; |
| Found: | C, 69.11; | H, 5.55; | N, 15.09. |

### Example 9

### (trans)-N˝-Cyano-N-(3,4-dihydro-6-(phenylethynyl)-2H-1-benzopyran-4-yl)-N′-phenylguanidine

### A. 2,2-Dimethyl-6-(phenylethynyl)-2H-1-benzopyran

To a solution of the title B compound from Example 8 (1.69 g, 5.91 mmol) and phenylacetylene (2.0 mL, 18.1 mmol) in diethylamine (30 mL) at room temperature were added bis(triphenylphosphine)palladium(II)chloride (0.40 g, 0.572 mmol) and copper(I) iodide (0.22 g, 1.41 mmol) under argon atmosphere. After stirring for 1 hour at room temperature, the reaction mixture was concentrated under reduced pressure. The residue was dissolved in ethyl acetate and the insoluble material was filtered. The filtrate was concentrated *in vacuo*. The residue was again dissolved in toluene/hexane (1:10) and the insoluble material was filtered. The filtrate was concentrated *in vacuo* and the crude product was purified by flash chromatography on silica gel eluting with toluene/hexane (1:10) to give the title compound as an oil (1.43 g, 5.49 mmol) in 92% yield: ¹H NMR (CDCl₃) δ 7.47-7.50 (m, 2H), 7.24-7.31 (m, 4H), 7.14 (d, J = 2.4 Hz, 1H), 6.72 (d, J = 8.2 Hz, 1H), 6.26 (d, J = 10.0 Hz, 1H), 5.58 (d, J = 9.4 Hz, 1H), 1.40 (s, 6H); ¹³C NMR (CDCl₃) δ 153.2, 132.5, 131.3, 131.2, 129.5, 128.2, 127.8, 123.6, 121.6, 121.1, 116.4, 115.2, 89.4, 87.8, 76.7, 28.0.

### B. 2,2-Dimethyl-6-(phenylethynyl)-2H-oxireno-(c)-(1)-benzopyran

To a solution of the title A compound (1.14, 4.38 mmol) and sodium bicarbonate (1.86 g, 22.1 mmol) in methylene chloride (15 mL) and water (15 mL) at 0° was added 3-chloroperoxybenzoic acid (1.21 g of 80-85% purity, 5.62 mmol). After 5 minutes, the ice bath was removed and the reaction mixture was stirred at room temperature for 8 hours. It was diluted with methylene chloride and the organic layer was taken. It was washed with water followed by brine and dried over magnesium sulfate. The solvent was concentrated *in vacuo* and the material was purified by flash chromatography on silica gel eluting with hexane/ethyl acetate (10:1) to give recovered starting material (0.17 g) and the title compound as an oil (0.74 g, 2.68 mmol) in 61% yield: ¹H NMR (CDCl₃) δ 7.42-7.64 (m, 7H), 6.90 (d, J = 8.8 Hz, 1H), 3.99 (d, J = 4.7 Hz, 1H), 3.60 (d, J = 4.7 Hz, 1H), 1.69 (s, 3H), 1.38 (s, 3H); ¹³C NMR (CDCl₃) δ 152.8, 133.7, 132.9, 131.4, 128.3, 128.0, 123.4, 120.2, 118.2, 115.9, 88.9, 88.3, 73.6 62.5, 50.6, 48.2, 22.7.

### C. (trans)-4-Amino-3,4-dihydro-2,2-dimethyl-6-(phenylethynyl)-2H-1-benzopyran-3-ol

A solution of the title B compound (0.71 g, 2.55 mmol) in absolute ethanol (20 mL) and concentrated aqueous ammonium hydroxide (40 mL) was stirred for 7 days and the solvents were removed *in vacuo*. The crude product was triturated with hexane and diisopropyl ether to give the title compound as a white solid (0.64 g, 2.18 mmol) in 86% yield, m.p. 162-164°C; ¹H NMR (CDCl₃) δ 7.36-7.67 (m, 7H), 6.86 (d, J = 8.2 Hz, 1H), 3.78 (d, J = 10.0 Hz, 1H), 3.48 (d, J = 10.0 Hz, 1H), 2.51 (br s, 3H), 1.62 (s, 3H), 1.32 (s, 3H); ¹³C NMR (CDCl₃) δ 152.7, 132.1, 131.4, 130.4, 128.3, 128.0, 125.6, 122.8, 117.3, 115.0, 88.6, 87.1, 78.5, 76.0, 51.2, 26.9, 18.7.

| Analysis calc'd for C₁₉H₁₉O₂N·0.28 H₂O: | | | |
|---|---|---|---|
| | C, 76.46; | H, 6.61; | N, 4.69; |
| Found: | C, 76.39; | H, 6.52; | N, 4.76. |

### D. (trans)-N˝-Cyano-N-(3,4-dihydro-6-(phenylethynyl)-2H-1-benzopyran-4-yl)-N′-phenylguanidine

To a solution of the title C compound (0.64 g, 2.18 mmol) and N-cyano-N′-phenylthiourea (0.56 g, 3.16 mmol) in dimethylformamide (16 mL) was added 1-(3-dimethylaminopropyl)-2-ethyl-carbodiimide hydrochloride (0.60 g, 3.49 mmol) at room temperature. The reaction mixture was stirred at room temperature for 2 days and the solvent was removed *in vacuo*. The residue was partitioned between ethyl acetate and water. The organic layer was taken and it was washed with water followed by brine, dried over sodium sulfate and concentrated *in vacuo*. The residue was purified by flash chromatography on silica gel eluting with hexane/ethyl acetate/ethanol (10:10:1) to give a partially purified material. This material was triturated with diisopropyl ether to give the title compound as a white solid (0.46 g, 1.05 mmol) in 48% yield, m.p. 175-177°C: ¹H NMR (DMSO-d₆) δ 9.42 (s, 1H), 7.82 (d, J = 8.8 Hz, 1H), 7.20-7.75 (m, 14H), 6.88 (d, J = 9.4 Hz, 1H), 5.59 (br s, 1H), 5.02 (dd, J₁ = 8.8, J₂ = 9.4 Hz, 1H), 3.80 (br d, J = 9.4 Hz, 1H), 1.50 (s, 3H), 1.27 (s, 3H); ¹³C NMR (DMSO-d₆) δ 159.5, 153.2, 138.0, 132.2, 131.6, 131.3. 129.3, 129.0, 128.0, 124.9, 124.1, 123.7, 122.9, 117.4, 114.3, 89.7, 88.3, 79.9, 71.6, 52.5, 27.1, 18.8.

## Claims (Claims for the following Contracting State(s): AT, BE, CH, DE, DK, FR, GB, IT, LI, LU, NL, SE)

1. A compound of the formula 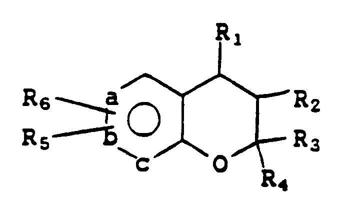 wherein a, b, and c are all =CH- or one of a, b and c is nitrogen or =N(O)- and the others are =CH-;
R₁ is 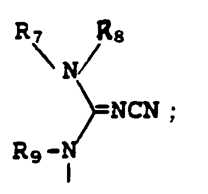 R₂ is hydrogen, hydroxy or 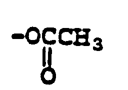 R₃ and R₄ are each independently hydrogen, alkyl or arylalkyl, or, R₃ and R₄ taken together with the carbon atom to which they are attached form a 5- to 7-membered carbocyclic ring;
R₅ is selected from H, alkyl, haloalkyl, alkenyl, alkynyl, cycloalkyl, arylalkyl, cycloalkylalkyl, -CN, -NO₂, -COR, -COOR, -CONHR, -CONR₂, -CF₃,-S-alkyl, -SOalkyl, -SO₂alkyl, 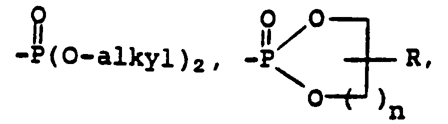 halogen, amino, substituted amino, O-alkyl, OCF₃, OCH₂CF₃, -OCOalkyl, -OCONRalkyl, -NRCOalkyl, NRCOOalkyl, and NRCONR₂ wherein R in each of the above groups can be hydrogen, alkyl, aryl, arylalkyl, cycloalkyl, or (cycloalkyl)alkyl;
R₆ is selected from H, alkyl, OH, O-alkyl, amino, substituted amino, CN, and NO₂;
R₇ is selected from aryl, heterocyclo, and (heterocyclo)alkyl;
R₈ is selected from hydrogen, alkyl, alkenyl, aryl, and arylalkyl;
R₉ is selected from hydrogen, alkyl, alkenyl, aryl, arylalkyl, cycloalkyl and cycloalkylalkyl; and
n is 1, 2 or 3;
wherein:
"alkyl" means a C₁ to C₈ alkyl group;
"alkenyl" means a C₂ to C₈ alkenyl group;
"cycloalkyl" means a C₃ to C₇ cycloalkyl group;
"halogen" and "halo" mean chloro, bromo or fluoro;
"aryl" means phenyl, 1-naphthyl, 2-naphthyl or mono substituted phenyl, 1-naphthyl, or 2-naphthyl wherein said substituent is alkyl of 1 to 4 carbons, alkylthio of 1 to 4 carbons, alkoxy of 1 to 4 carbons, halo, nitro, cyano, hydroxy, amino, -NH-alkyl wherein alkyl is of 1 to 4 carbons, -N(alkyl)₂ wherein alkyl is of 1 to 4 carbons,
-CF₃, -OCHF₂, 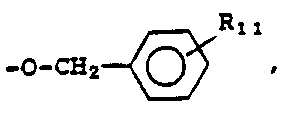 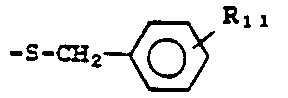 (wherein R₁₁ is hydrogen, alkyl of 1 to 4 carbons, alkoxy of 1 to 4 carbons, alkylthio of 1 to 4 carbons, halo, hydroxy or CF₃), -O-CH₂-cycloalkyl, or -S-CH₂- cycloalkyl, and di-substituted phenyl, 1-naphthyl, 2-naphthyl wherein said substituents are selected from methyl, methoxy, methylthio, halo, CF₃, nitro, amino, and OCHF₂;
"heterocyclo" means (a) fully saturated or unsaturated rings of 5 or 6 atoms containing one or two O and S atoms and/or one to four N atoms provided that the total number of hetero atoms in the ring is 4 or less, (b) bicyclic rings wherein a five or six membered ring containing O, S and N atoms as defined above is fused to a benzene ring, and (c) such monocyclic and bicyclic rings wherein an available carbon atom is substituted with a lower alkyl of 1 to 4 carbons, lower alkylthio of 1 to 4 carbons, lower alkoxy of 1 to 4 carbons, halo, nitro, keto, cyano, hydroxy, amino, -NH-alkyl wherein alkyl is of 1 to 4 carbons, -N(alkyl)₂ wherein alkyl is of 1 to 4 carbons, CF₃, or OCHF₂ or such monocyclic and bicyclic rings wherein two or three available carbons have substituents selected from methyl, methoxy, methylthio, halo, CF₃, nitro, hydroxy, amino and OCHF₂; the rings in all cases being attached by an available carbon atom; and
"substituted amino" means a group of the formula -NZ₁Z₂ wherein Z₁ is hydrogen, alkyl, cycloalkyl, aryl, arylalkyl, or cycloalkylalkyl and Z₂ is alkyl, cycloalkyl, aryl, arylalkyl, or cycloalkylalkyl or Z₁ and Z₂ taken together with the nitrogen atom to which they are attached are 1-pyrrolidinyl, 1-piperidinyl, 1-azepinyl, 4-morpholinyl, 4-thiamorpholinyl, 1-piperazinyl, 4-alkyl-1-piperazinyl, 4-arylalkyl-1-piperazinyl, 4-diarylalkyl-1-piperazinyl, or 1-pyrrolidinyl, 1-piperidinyl, or 1-azepinyl substituted with alkyl, alkoxy, alkylthio, halo, trifluoromethyl or hydroxy.

2. A compound in accordance with claim 1 wherein
a is nitrogen or =CR₅-;
b and c are each =CH-;
R₂ is hydroxy or -OCOCH₃;
R₃ and R₄ are each alkyl;
R₅ is an electron withdrawing group;
R₆ is hydrogen, alkyl, O-alkyl, or amino;
R₇ is aryl;
R₈ is hydrogen;
R₉ is hydrogen or alkyl; and
n is 1 or 2.

3. A compound in accordance with claim 1 wherein
a is nitrogen or =CR₅-;
b and c are each =CH-;
R₂ is transhydroxy;
R₃ and R₄ are each methyl;
R₅ is -CN or -NO₂;
R₆ is hydrogen;
R₇ is phenyl or monosubstituted phenyl wherein the substituent is nitro, halo, -CF₃, alkyl, cyano or methoxy;
R₈ is hydrogen;
R₉ is hydrogen; and
n is 1.

4. (T*rans*)-N''-cyano-N-(6-cyano-3,4-dihydro-3-hydroxy-2,2-dimethyl-2H-1-benzopyran-4-yl)-N'-phenyl guanidine.

5. (T*rans*)-N''-cyano-N-(3,4-dihydro-3-hydroxy-2,2-dimethyl-2H-pyrano[3,2-c]pyridin-4-yl)-phenylguanidine.

6. (3S-T*rans*)-N''-cyano-N-(6-cyano-3,4-dihydro-3-hydroxy-2,2-dimethyl-2H-1-benzopyran-4-yl)-N'-phenylguanidine.

7. (3R-T*rans*)-N''-cyano-N-(6-cyano-3,4-dihydro-3-hydroxy-2,2-dimethyl-2H-1-benzopyran-4-yl)-N'-phenylguanidine.

8. (T*rans*)-N''-cyano-N-(6-cyano-3,4-dihydro-3-hydroxy-2,2-dimethyl-2H-1-benzopyran-4-yl)-N'-(4-pyridinylmethyl)guanidine.

9. (T*rans*)-N''-cyano-N-(6-cyano-3,4-dihydro-3-hydroxy-2,2-dimethyl-2H-1-benzopyran-4-yl)-N'-(3-pyridinylmethyl)guanidine.

10. (T*rans*)-N''-cyano-N-(6-ethynyl-3,4-dihydro-3-hydroxy-2,2-dimethyl-2H-1-benzopyran-4-yl)-N'-phenylguanidine.

11. (T*rans*)-N''-cyano-N-(3,4-dihydro-6-(phenylethynyl)-2H-1-benzopyran-4-yl)-N'-phenylguanidine.

12. A compound in accordance with claim 1 having the structure

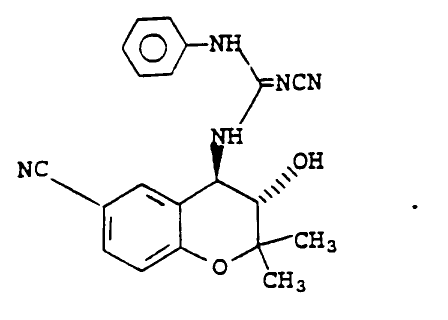

13. Use of a compound as defined in any preceding claim for the manufacture of a medicament for the treatment of myocardial ischemia in a mammalian specie.

14. The use as defined in claim 13 wherein said compound has the structure

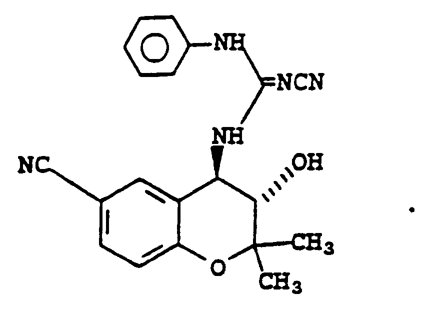

15. Use of a compound as defined in any one of claims 1-12 for the manufacture of a medicament for the treatment of hypertension in a mammalian specie.

16. A compound in accordance with claim 1, wherein
R₅ is selected from H, alkyl, haloalkyl, alkenyl, alkynyl, cycloalkyl, arylalkyl, cycloalkylalkyl, -CN, -NO₂, -COR, -COOR, -CONHR, -CONR₂, -CF₃, S-alkyl, -SOalkyl, -SO₂alkyl, halogen, amino, substituted amino, O-alkyl, -OCOalkyl, -OCONRalkyl, -NRCOalkyl, NRCOOalkyl,and NRCONR₂ wherein R in each of the above groups can be hydrogen, alkyl, aryl, arylalkyl, cycloalkyl, or cycloalkylalkyl.

17. A compound according to any one of claims 1-12 and 16 for use in treatments involving potassium channel activating activity.

18. Use of a compound according to any one of claims 1-12 and 16 for the manufacture of a medicament for treatment of arrythmia.

19. Use of a compound according to any one of claims 1-12 and 16 for the manufacture of a medicament for treatment of ischemia.

20. Use of a compound according to any one of claims 1-12 and 16 for the manufacture of a medicament for treatment of hypertension.

## Claims (Claims for the following Contracting State(s): ES, GR)

1. A process for preparing a compound of the formula 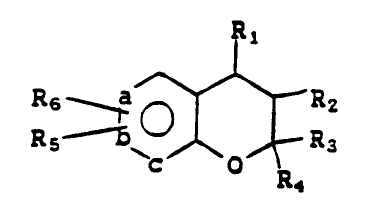 wherein a, b, and c are all =CH- or one of a, b and c is nitrogen or =N(O)- and the others are =CH-;
R₁ is 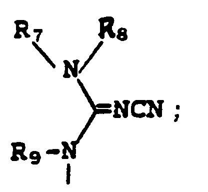 R₂ is hydrogen, hydroxy or 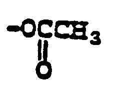 R₃ and R₄ are each independently hydrogen, alkyl or arylalkyl, or, R₃ and R₄ taken together with the carbon atom to which they are attached form a 5- to 7-membered carbocyclic ring;
R₅ is selected from H, alkyl, haloalkyl, alkenyl, alkynyl, cycloalkyl, arylalkyl, cycloalkylalkyl, -CN, -NO₂, -COR, -COOR, -CONHR, -CONR₂, -CF₃,-S-alkyl, -SOalkyl, -SO₂alkyl, 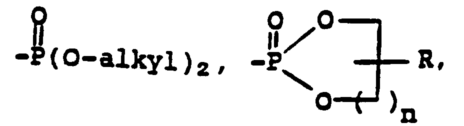 halogen, amino, substituted amino, O-alkyl, OCF₃, OCH₂CF₃, -OCOalkyl, -OCONRalkyl, -NRCOalkyl, NRCOOalkyl, and NRCONR₂ wherein R in each of the above groups can be hydrogen, alkyl, aryl, arylalkyl, cycloalkyl, or (cycloalkyl)alkyl;
R₆ is selected from H, alkyl, OH, O-alkyl, amino, substituted amino, CN, and NO₂;
R₇ is selected from aryl, heterocyclo, and (heterocyclo)alkyl;
R₈ is selected from hydrogen, alkyl, alkenyl, aryl, and arylalkyl;
R₉ is selected from hydrogen, alkyl, alkenyl, aryl, arylalkyl, cycloalkyl and cycloalkylalkyl; and
n is 1, 2 or 3;
wherein:
"alkyl" means a C₁ to C₈ alkyl group;
"alkenyl" means a C₂ to C₈ alkenyl group;
"cycloalkyl" means a C₃ to C₇ cycloalkyl group;
"halogen" and "halo" mean chloro, bromo or fluoro;
"aryl" means phenyl, 1-naphthyl, 2-naphthyl or mono substituted phenyl, 1-naphthyl, or 2-naphthyl wherein said substituent is alkyl of 1 to 4 carbons, alkylthio of 1 to 4 carbons, alkoxy of 1 to 4 carbons, halo, nitro, cyano, hydroxy, amino, -NH-alkyl wherein alkyl is of 1 to 4 carbons, -N(alkyl)₂ wherein alkyl is of 1 to 4 carbons,
-CF₃, -OCHF₂, 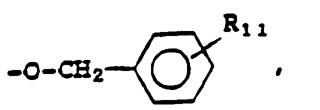 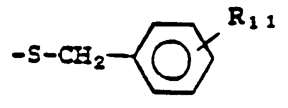 (wherein R₁₁ in hydrogen, alkyl of 1 to 4 carbons, alkoxy of 1 to 4 carbons, alkylthio of 1 to 4 carbons, halo, hydroxy or CF₃), -O-CH₂-cycloalkyl, or -S-CH₂- cycloalkyl, and di-substituted phenyl, 1-naphthyl, 2-naphthyl wherein said substituents are selected from methyl, methoxy, methylthio, halo, CF₃, nitro, amino, and OCHF₂;
"heterocyclo" means (a) fully saturated or unsaturated rings of 5 or 6 atoms containing one or two O and S atoms and/or one to four N atoms provided that the total number of hetero atoms in the ring is 4 or less, (b) bicyclic rings wherein a five or six membered ring containing O, S and N atoms as defined above is fused to a benzene ring, and (c) such monocyclic and bicyclic rings wherein an available carbon atom is substituted with a lower alkyl of 1 to 4 carbons, lower alkylthio of 1 to 4 carbons, lower alkoxy of 1 to 4 carbons, halo, nitro, keto, cyano, hydroxy, amino, -NH-alkyl wherein alkyl is of 1 to 4 carbons, -N(alkyl)₂ wherein alkyl is of 1 to 4 carbons, CF₃, or OCHF₂ or such monocyclic and bicyclic rings wherein two or three available carbons have substituents selected from methyl, methoxy, methylthio, halo, CF₃, nitro, hydroxy, amino and OCHF₂; the rings in all cases being attached by an available carbon atom; and
"substituted amino" means a group of the formula -NZ₁Z₂ wherein Z₁ is hydrogen, alkyl, cycloalkyl, aryl, arylalkyl, or cycloalkylalkyl and Z₂ is alkyl, cycloalkyl, aryl, arylalkyl, or cycloalkylalkyl or Z₁ and Z₂ taken together with the nitrogen atom to which they are attached are 1-pyrrolidinyl, 1-piperidinyl, 1-azepinyl, 4-morpholinyl, 4-thiamorpholinyl, 1-piperazinyl, 4-alkyl-1-piperazinyl, 4-arylalkyl-1-piperazinyl, 4-diarylalkyl-1-piperazinyl, or 1-pyrrolidinyl, 1-piperidinyl, or 1-azepinyl substituted with alkyl, alkoxy, alkylthio, halo, trifluoromethyl or hydroxy;
which process comprises
(i) treating a thiourea of the formula 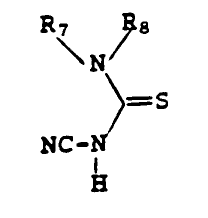 with an amine of the formula 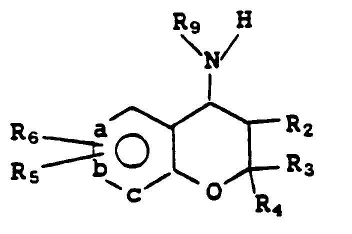 in the presence of a carbodiimide and an acid source in an organic solvent; or
(ii) heating a thiourea of the formula 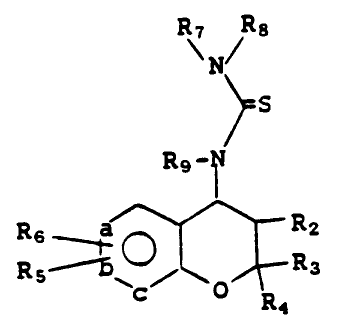 with monosodium cyanamide in the presence of a carbodiimide in an organic solvent; or
(iii) reacting a compound of the formula 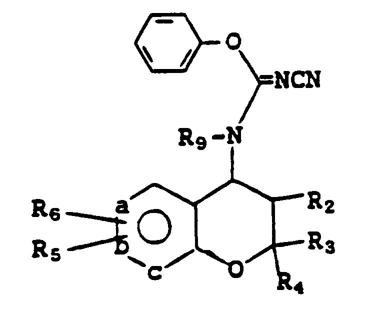 with an amine of the formula
R₇R₈NH
in a polar solvent.

2. A process in accordance with claim 1 wherein
a is nitrogen or =CR₅-;
b and c are each =CH-;
R₂ is hydroxy or -OCOCH₃;
R₃ and R₄ are each alkyl;
R₅ is an electron withdrawing group;
R₆ is hydrogen, alkyl, O-alkyl, or amino;
R₇ is aryl;
R₆ is hydrogen;
R₉ is hydrogen or alkyl; and
n is 1 or 2.

3. A process in accordance with claim 1 wherein
a is nitrogen or =CR₅-;
b and c are each =CH-;
R₂ is transhydroxy;
R₃ and R₄ are each methyl;
R₅ is -CN or -NO₂;
R₆ is hydrogen;
R₇ is phenyl or monosubstituted phenyl wherein the substituent is nitro, halo, -CF₃, alkyl, cyano or methoxy;
R₈ is hydrogen;
R₉ is hydrogen; and
n is 1.

4. A process in accordance with claim 1 wherein the product compound is (trans)-N''-cyano-N-(6-cyano-3,4-dihydro-3-hydroxy-2,2-dimethyl-2H-1-benzopyran-4-yl)-N'-phenyl guanidine.

5. A process in accordance with claim 1 wherein the product compound is (trans)-N''-cyano-N-(3,4-dihydro-3-hydroxy-2,2-dimethyl-2H-pyrano[3,2-c]pyridin-4-yl)-phenylguanidine.

6. A process in accordance with claim 1 wherein the product compound is (3S-trans) -N''-cyano-N-(6-cyano-3,4-dihydro-3-hydroxy-2,2-dimethyl-2H-1-benzopyran-4-yl)-N'-phenylguanidine.

7. A process in accordance with claim 1 wherein the product compound is (3R-trans) -N''-cyano-N-(6-cyano-3,4-dihydro-3-hydroxy-2,2-dimethyl-2H-1-benzopyran-4-yl)-N'-phenylguanidine.

8. A process in accordance with claim 1 wherein the product compound is (trans)-N''-cyano-N-(6-cyano-3,4-dihydro-3-hydroxy-2,2-dimethyl-2H-1-benzopyran-4-yl)-N'-(4-pyridinylmethyl)guanidine.

9. A process in accordance with claim 1 wherein the product compound is (trans)-N''-cyano-N-(6-cyano-3,4-dihydro-3-hyoroxy-2,2-dimethyl-2H-1-benzopyran-4-yl)-N'-(3-pyridinylmethyl)guanidine.

10. A process in accordance with claim 1 wherein the product compound is (trans)-N''-cyano-N-(6-ethynyl-3,4-dihydro-3-hydroxy-2,2-dimethyl-2H-1-benzopyran-4-yl)-N'-phenylguanidine.

11. A process in accordance with claim 1 wherein the product compound is (trans)-N''-cyano-N-(3,4-dihydro-6-(phenylethynyl)-2H-1-benzopyran-4-yl)-N'-phenylguanidine.

12. A process in accordance with claim 1 wherein the product compound has the structure

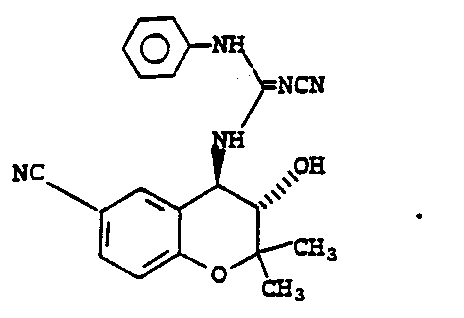

13. A process in accordance with claim 1 wherein
R₅ is selected from H, alkyl, haloalkyl, alkenyl, alkynyl, cycloalkyl, arylalkyl, cycloalkylalkyl, -CN, -NO₂, -COR, -COOR, -CONHR, -CONR₂, -CF₃, S-alkyl, -SOalkyl, -SO₂alkyl, halogen, amino, substituted amino, O-alkyl, -OCOalkyl, -OCONRalkyl, -NRCOalkyl, NRCOOalkyl, and NRCONR₂ wherein R in each of the above groups can be hydrogen, alkyl, aryl, arylalkyl, cycloalkyl, or cycloalkylalkyl.

## Patentansprüche (Patentansprüche für folgende(n) Vertragsstaat(en): AT, BE, CH, DE, DK, FR, GB, IT, LI, LU, NL, SE)

1. Verbindung der Formel 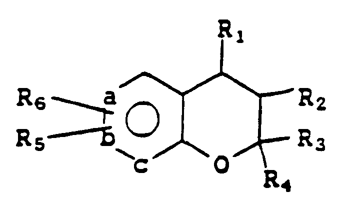 in der a, b und c eine Gruppe der Formel =CH- sind, oder eines von a, b und c ein Stickstoffatom oder eine Gruppe der Formel =N(O)- ist, und die anderen eine Gruppe der Formel =CH-sind;
R₁ ein Rest der Formel 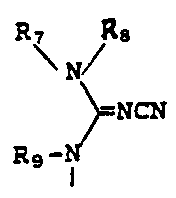 ist;
R₂ ein Wasserstoffatom, eine Hydroxylgruppe oder eine Gruppe der Formel 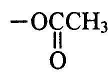 ist;
R₃ und R₄ jeweils unabhängig voneinander ein Wasserstoffatom, Alkyl- oder Arylalkylrest sind, oder R₃ und R₄ zusammen mit dem Kohlenstoffatom, an das sie gebunden sind, einen 5- bis 7-gliedrigen carbocyclischen Ring bilden;
R₅ aus einem Wasserstoffatom, Alkyl-, Halogenalkyl-, Alkenyl-, Alkinyl-, Cycloalkyl-, Arylalkyl-, Cycloalkylalkylrest, einer Gruppe der Formel -CN, -NO₂, einem Rest der Formel -COR, -COOR, -CONHR, -CONR₂, einer Gruppe der Formel -CF₃, einem Rest der Formel -S-Alkyl, -SO-Alkyl, -SO₂-Alkyl, 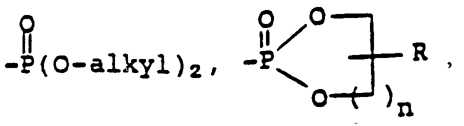 einem Halogenatom, einer Aminogruppe, einem substituierten Aminorest, einem Rest der Formel -O-Alkyl, einer Gruppe der Formel -OCF₃, -OCH₂CF₃, einem Rest der Formel -OCO-Alkyl, -OCONR-Alkyl, -NRCO-Alkyl, -NRCOO-Alkyl und -NRCONR₂ ausgewählt ist, wobei der Rest R in jedem der vorstehenden Reste ein Wasserstoffatom, Alkyl-, Aryl-, Arylalkyl-, Cycloalkyl- oder (Cycloalkyl)alkylrest sein kann;
R₆ aus einem Wasserstoffatom, Alkylrest, einer OH-Gruppe, einem Rest der Formel -O-Alkyl, einer Aminogruppe, einem substituierten Aminorest, einer Gruppe der Formel -CN und -NO₂ ausgewählt ist;
R₇ aus einem Aryl-, Heterocyclo- und (Heterocyclo)alkylrest ausgewählt ist;
R₈ aus einem Wasserstoffatom, Alkyl-, Alkenyl-, Aryl- und Arylalkylrest ausgewählt ist;
R₉ aus einem Wasserstoffatom, Alkyl-, Alkenyl-, Aryl-, Arylalkyl-, Cycloalkyl und Cycloalkylalkylrest ausgewählt ist; und
n 1, 2 oder 3 ist;
wobei:
"Alkylrest" einen C₁- bis C₈-Alkylrest bedeutet;
"Alkenylrest" einen C₂- bis C₈-Alkenylrest bedeutet;
"Cycloalkylrest" einen C₃- bis C₇-Cycloalkylrest bedeutet;
"Halogenatom" und "Halogen" ein Chlor-, Brom- oder Fluoratom bedeuten;
"Arylrest" eine Phenyl-, 1-Naphthyl- oder 2-Naphthylgruppe oder einen monosubstituierten Phenyl-, 1-Naphthyl- oder 2-Naphthylrest, wobei der Substituent ein Alkylrest aus 1 bis 4 Kohlenstoffatomen, ein Alkylthiorest aus 1 bis 4 Kohlenstoffatomen, ein Alkoxyrest aus 1 bis 4 Kohlenstoffatomen, ein Halogenatom, eine Nitro-, Cyano-, Hydroxyl-, Aminogruppe, ein Rest der Formel -NH-Alkyl, wobei der Alkylrest aus 1 bis 4 Kohlenstoffatomen besteht, ein Rest der Formel -N(Alkyl)₂, wobei der Alkylrest aus 1 bis 4 Kohlenstoffatomen besteht, eine Gruppe der Formel -CF₃, -OCHF₂, ein Rest der Formel, 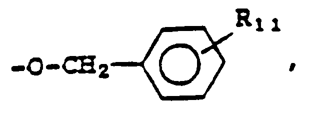 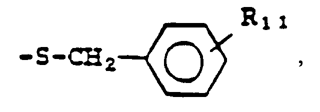 (in denen R₁₁ ein Wasserstoffatom, Alkylrest aus 1 bis 4 Kohlenstoffatomen, Alkoxyrest aus 1 bis 4 Kohlenstoffatomen, Alkylthiorest aus 1 bis 4 Kohlenstoffatomen, Halogenatom, eine Hydroxylgruppe oder eine Gruppe der Formel -CF₃ ist), ein Rest der Formel -O-CH₂-Cycloalkyl oder -S-CH₂-Cycloalkyl ist, und einen disubstituierten Phenyl-, 1-Naphthyl- oder 2-Naphthylrest bedeutet, wobei die Substituenten aus einer Methyl-, Methoxy-, Methylthiogruppe, einem Halogenatom, einer Gruppe der Formel -CF₃, Nitro-, Aminogruppe und einer Gruppe der Formel -OCHF₂ ausgewählt sind;
"Heterocyclorest" (a) vollständig gesättigte oder ungesättigte Ringe aus 5 oder 6 Atomen, die 1 oder 2 Sauerstoff- und Schwefelatome und/oder 1 bis 4 Stickstoffatome enthalten, mit der Maßgabe, daß die Gesamtzahl der Heteroatome im Ring 4 oder weniger ist, (b) bicyclische Ringe, in denen ein 5- oder 6-gliedriger Ring mit Sauerstoff-, Schwefel- und Stickstoffatomen, wie vorstehend definiert, an einen Benzolring kondensiert ist, und (c) die monocyclischen und bicyclischen Ringe, in denen ein verfügbares Kohlenstoffatom mit einem Niederalkylrest aus 1 bis 4 Kohlenstoffatomen, Niederalkylthiorest aus 1 bis 4 Kohlenstoffatomen, Niederalkoxyrest aus 1 bis 4 Kohlenstoffatomen, einem Halogenatom, einer Nitro-, Keto-, Cyano-, Hydroxyl-, Aminogruppe, einem Rest der Formel -NH-Alkyl, wobei der Alkylrest aus 1 bis 4 Kohlenstoffatomen besteht, einem Rest der Formel -N(Alkyl)₂, wobei der Alkylrest aus 1 bis 4 Kohlenstoffatomen besteht, einer Gruppe der Formel -CF₃ oder -OCHF₂ substituiert ist, oder die monocyclischen und bicyclischen Ringe bedeutet, in denen zwei oder drei verfügbare Kohlenstoffatome Substituenten aufweisen, die aus einer Methyl-, Methoxy-, Methylthiogruppe, einem Halogenatom, einer Gruppe der Formel -CF₃, Nitro-, Hydroxyl-, Aminogruppe und einer Gruppe der Formel -OCHF₂ ausgewählt sind, wobei die Ringe in allen Fällen über ein verfügbares Kohlenstoffatom gebunden sind; und
"Substituierter Aminorest" einen Rest der Formel -NZ₁Z₂ bedeutet, in der Z₁ ein Wasserstoffatom, Alkyl-, Cycloalkyl-, Aryl-, Arylalkyl- oder Cycloalkylalkylrest ist, und Z₂ ein Alkyl-, Cycloalkyl-, Aryl-, Arylalkyl- oder Cycloalkylalkylrest ist, oder Z₁ und Z₂ zusammen mit dem Stickstoffatom, an das sie gebunden sind, eine 1-Pyrrolidinyl-, 1-Piperidinyl-, 1-Azepinyl-, 4-Morpholinyl-, 4-Thiamorpholinyl-, 1-Piperazinyl-, 4-Alkyl-1-piperazinyl-, 4-Arylalkyl-1-piperazinyl-, 4-Diarylalkyl-1-piperazinylgruppe oder ein mit einem Alkyl-, Alkoxy-, Alkylthiorest, Halogenatom, einer Trifluormethyl- oder Hydroxylgruppe substituierter 1-Pyrrolidinyl-, 1-Piperidinyl- oder 1-Azepinylrest sind.

2. Verbindung gemäß Anspruch 1, in der
a ein Stickstoffatom oder ein Rest der Formel =CR₅- ist;
b und c jeweils eine Gruppe der Formel =CH- sind;
R₂ eine Hydroxylgruppe oder eine Gruppe der Formel -OCOCH₃ ist;
R₃ und R₄ jeweils ein Alkylrest sind;
R₅ eine elektronenziehende Gruppe ist;
R₆ ein Wasserstoffatom, Alkylrest, ein Rest der Formel -O-Alkyl oder eine Aminogruppe ist;
R₇ ein Arylrest ist;
R₈ ein Wasserstoffatom ist;
R₉ ein Wasserstoffatom oder ein Alkylrest ist; und
n 1 oder 2 ist.

3. Verbindung gemäß Anspruch 1, in der
a ein Stickstoffatom oder ein Rest der Formel =CR₅- ist;
b und c jeweils eine Gruppe der Formel =CH- sind;
R₂ eine trans-Hydroxylgruppe ist;
R₃ und R₄ jeweils eine Methylgruppe sind;
R₅ eine Gruppe der Formel -CN oder -NO₂ ist;
R₆ ein Wasserstoffatom ist;
R₇ eine Phenylgruppe oder ein monosubstituierter Phenylrest ist, wobei der Substituent eine Nitrogruppe, ein Halogenatom, eine Gruppe der Formel -CF₃, ein Alkylrest, eine Cyano- oder Methoxygruppe ist;
R₈ ein Wasserstoffatom ist;
R₉ ein Wasserstoffatom ist; und
n 1 ist.

4. (*trans*)-N''-Cyano-N-(6-cyano-3,4-dihydro-3-hydroxy-2,2-dimethyl-2H-1-benzopyran-4-yl)-N'-phenylguanidin.

5. (*trans*)-N''-Cyano-N-(3,4-dihydro-3-hydroxy-2,2-dimethyl-2H-pyrano[3,2-c]pyridin-4-yl)phenylguanidin.

6. (3S-*trans*)-N''-Cyano-N-(6-cyano-3,4-dihydro-3-hydroxy-2,2-dimethyl-2H-1-benzopyran-4-yl)-N'-phenylguanidin.

7. (3R-*trans*)-N''-Cyano-N-(6-cyano-3,4-dihydro-3-hydroxy-2,2-dimethyl-2H-1-benzopyran-4-yl)-N'-phenylguanidin.

8. (*trans*)-N''-Cyano-N-(6-cyano-3,4-dihydro-3-hydroxy-2,2-dimethyl-2H-1-benzopyran-4-yl)-N'-(4-pyridinylmethyl)guanidin.

9. (*trans*)-N''-Cyano-N-(6-cyano-3,4-dihydro-3-hydroxy-2,2-dimethyl-2H-1-benzopyran-4-yl)-N'-(3-pyridinylmethyl)guanidin.

10. (*trans*)-N''-Cyano-N-(6-ethinyl-3,4-dihydro-3-hydroxy-2,2-dimethyl-2H-1-benzopyran-4-yl)-N'-phenylguanidin.

11. (*trans*)-N''-Cyano-N-(3,4-dihydro-6-(phenylethinyl)-2H-1-benzopyran-4-yl)-N'-phenylguanidin.

12. Verbindung gemäß Anspruch 1 mit der Struktur

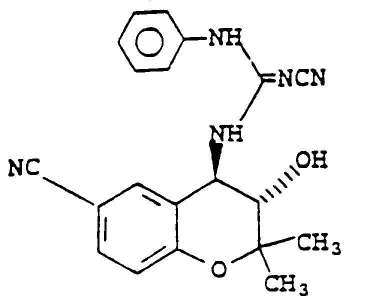

13. Verwendung einer Verbindung nach einem der vorstehenden Ansprüche zur Herstellung eines Arzneimittels zur Behandlung von myokardialer Ischämie in einer Säugerspezies.

14. Verwendung nach Anspruch 13, wobei die Verbindung die Struktur 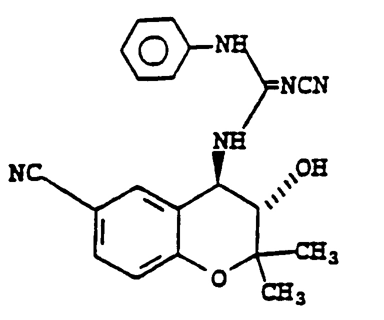 aufweist.

15. Verwendung einer Verbindung nach einem der Ansprüche 1 - 12 zur Herstellung eines Arzneimittels zur Behandlung von Hypertonie in einer Säugerspezies.

16. Verbindung gemäß Anspruch 1, wobei R₅ aus einem Wasserstoffatom, Alkyl-, Halogenalkyl-, Alkenyl-, Alkinyl-, Cycloalkyl-, Arylalkyl-, Cycloalkylalkylrest, einer Gruppe der Formel -CN, -NO₂, einem Rest der Formel -COR, -COOR, -CONHR, -CONR₂, einer Gruppe der Formel -CF₃ , einem Rest der Formel -S-Alkyl, -SO-Alkyl, -SO₂-Alkyl, einem Halogenatom, einer Aminogruppe, einem substituierten Aminorest, einem Rest der Formel -O-Alkyl, -OCO-Alkyl, -OCONR-Alkyl, -NRCO-Alkyl, -NRCOO-Alkyl und -NRCONR₂ ausgewählt ist, wobei der Rest R in jedem der vorstehenden Reste ein Wasserstoffatom, Alkyl-, Aryl-, Arylalkyl-, Cycloalkyl- oder (Cycloalkyl)alkylrest sein kann.

17. Verbindung gemäß einem der Ansprüche 1-12 und 16 zur Verwendung bei Behandlungen, die eine Kaliumkanal-aktivierende Aktivität umfassen.

18. Verwendung einer Verbindung gemäß einem der Ansprüche 1 - 12 und 16 zur Herstellung eines Arzneimittels zur Behandlung von Arrythmie.

19. Verwendung einer Verbindung gemäß einem der Ansprüche 1-12 und 16 zur Herstellung eines Arzneimittels zur Behandlung von Ischämie.

20. Verwendung einer Verbindung gemäß einem der Ansprüche 1-12 und 16 zur Herstellung eines Arzneimittels zur Behandlung von Hypertonie.

## Patentansprüche (Patentansprüche für folgende(n) Vertragsstaat(en): ES, GR)

1. Verfahren zur Herstellung einer Verbindung der Formel 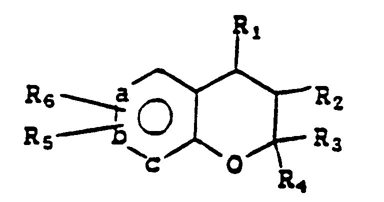 in der a, b und c eine Gruppe der Formel =CH- sind, oder eines von a, b und c ein Stickstoffatom oder eine Gruppe der Formel =N(O)- ist, und die anderen eine Gruppe der Formel =CH-sind;
R₁ ein Rest der Formel 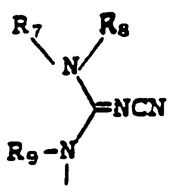 ist;
R₂ ein Wasserstoffatom, eine Hydroxylgruppe oder eine Gruppe der Formel 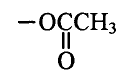 ist;
R₃ und R₄ jeweils unabhängig voneinander ein Wasserstoffatom, Alkyl- oder Arylalkylrest sind, oder R₃ und R₄ zusammen mit dem Kohlenstoffatom, an das sie gebunden sind, einen 5- bis 7-gliedrigen carbocyclischen Ring bilden;
R₅ aus einem Wasserstoffatom, Alkyl-, Halogenalkyl-, Alkenyl-, Alkinyl-, Cycloalkyl-, Arylalkyl-, Cycloalkylalkylrest, einer Gruppe der Formel -CN, -NO₂, einem Rest der Formel -COR, -COOR, -CONHR; -CONR₂, einer Gruppe der Formel -CF₃, einem Rest der Formel -S-Alkyl, -SO-Alkyl, -SO₂-Alkyl, 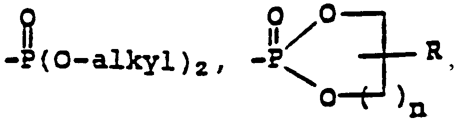 einem Halogenatom, einer Aminogruppe, einem substituierten Aminorest, einem Rest der Formel -O-Alkyl, einer Gruppe der Formel -OCF₃, -OCH₂CF₃, einem Rest der Formel -OCO-Alkyl, -OCONR-Alkyl, -NRCO-Alkyl, -NRCOO-Alkyl und -NRCONR₂ ausgewählt ist, wobei der Rest R in jedem der vorstehenden Reste ein Wasserstoffatom, Alkyl-, Aryl-, Arylalkyl-, Cycloalkyl- oder (Cycloalkyl)alkylrest sein kann;
R₆ aus einem Wasserstoffatom, Alkylrest, einer OH-Gruppe, einem Rest der Formel -O-Alkyl, einer Aminogruppe, einem substituierten Aminorest, einer Gruppe der Formel -CN- und -NO₂ ausgewählt ist;
R₇ aus einem Aryl-, Heterocyclo- und (Heterocyclo)alkylrest ausgewählt ist;
R₈ aus einem Wasserstoffatom, Alkyl-, Alkenyl-, Aryl- und Arylalkylrest ausgewählt ist;
R₉ aus einem Wasserstoffatom, Alkyl-, Alkenyl-, Aryl-, Arylalkyl-, Cycloalkyl- und Cycloalkylalkylrest ausgewählt ist; und
n 1, 2 oder 3 ist,
wobei:
"Alkylrest" einen C₁- bis C₈-Alkylrest bedeutet;
"Alkenylrest" einen C₂- bis C₈-Alkenylrest bedeutet;
"Cycloalkylrest" einen C₃- bis C₇-Cycloalkylrest bedeutet;
"Halogenatom" und "Halogen" ein Chlor-, Brom- oder Fluoratom bedeuten;
"Arylrest" eine Phenyl-, 1-Naphthyl- oder 2-Naphthylgruppe oder einen monosubstituierten Phenyl-, 1-Naphthyl- oder 2-Naphthylrest, wobei der Substituent ein Alkylrest aus 1 bis 4 Kohlenstoffatomen, ein Alkylthiorest aus 1 bis 4 Kohlenstoffatomen, ein Alkoxyrest aus 1 bis 4 Kohlenstoffatomen, ein Halogenatom, eine Nitro-, Cyano-, Hydroxyl-, Aminogruppe, ein Rest der Formel -NH-Alkyl, wobei der Alkylrest aus 1 bis 4 Kohlenstoffatomen besteht, ein Rest der Formel -N(Alkyl)₂, wobei der Alkylrest aus 1 bis 4 Kohlenstoffatomen besteht, eine Gruppe der Formel -CF₃, -OCHF₂, ein Rest der Formel, 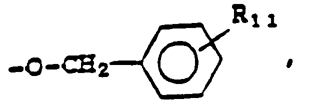 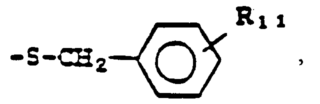 (in denen R₁₁ ein Wasserstoffatom, Alkylrest aus 1 bis 4 Kohlenstoffatomen, Alkoxyrest aus 1 bis 4 Kohlenstoffatomen, Alkylthiorest aus 1 bis 4 Kohlenstoffatomen, Halogenatom, eine Hydroxylgruppe oder eine Gruppe der Formel -CF₃ ist), ein Rest der Formel -O-CH₂-Cycloalkyl oder -S-CH₂-Cycloalkyl ist, und einen disubstituierten Phenyl-, 1-Naphthyl- oder 2-Naphthylrest bedeutet, wobei die Substituenten aus einer Methyl-, Methoxy-, Methylthiogruppe, einem Halogenatom, einer Gruppe der Formel -CF₃, Nitro-, Aminogruppe und einer Gruppe der Formel -OCHF₂ ausgewählt sind.
"Heterocyclorest" (a) vollständig gesättigte oder ungesättigte Ringe aus 5 oder 6 Atomen, die 1 oder 2 Sauerstoff- und Schwefelatome und/oder 1 bis 4 Stickstoffatome enthalten, mit der Maßgabe, daß die Gesamtzahl der Heteroatome im Ring 4 oder weniger ist, (b) bicyclische Ringe, in denen ein 5- oder 6-gliedriger Ring mit Sauerstoff-, Schwefel- und Stickstoffatomen, wie vorstehend definiert, an einen Benzolring kondensiert ist, und (c) die monocyclischen und bicyclischen Ringe, in denen ein verfügbares Kohlenstoffatom mit einem Niederalkylrest aus 1 bis 4 Kohlenstoffatomen, Niederalkylthiorest aus 1 bis 4 Kohlenstoffatomen, Niederalkoxyrest aus 1 bis 4 Kohlenstoffatomen, einem Halogenatom, einer Nitro-, Keto-, Cyano-, Hydroxyl-, Aminogruppe, einem Rest der Formel -NH-Alkyl, wobei der Alkylrest aus 1 bis 4 Kohlenstoffatomen besteht, einem Rest der Formel -N(Alkyl)₂, wobei der Alkylrest aus 1 bis 4 Kohlenstoffatomen besteht, einer Gruppe der Formel -CF₃ oder -OCHF₂ substituiert ist, oder die monocyclischen und bicyclischen Ringe bedeutet, in denen zwei oder drei verfügbare Kohlenstoffatome Substituenten aufweisen, die aus einer Methyl-, Methoxy-, Methylthiogruppe, einem Halogenatom, einer Gruppe der Formel -CF₃, Nitro-, Hydroxyl-, Aminogruppe und einer Gruppe der Formel -OCHF₂ ausgewählt sind, wobei die Ringe in allen Fällen über ein verfügbares Kohlenstoffatom gebunden sind; und
"Substituierter Aminorest" einen Rest der Formel -NZ₁Z₂ bedeutet, in der Z₁ ein Wasserstoffatom, Alkyl-, Cycloalkyl-, Aryl-, Arylalkyl- oder Cycloalkylalkylrest ist, und Z₂ ein Alkyl-, Cycloalkyl-, Aryl-, Arylalkyl- oder Cycloalkylalkylrest ist, oder Z₁ und Z₂ zusammen mit dem Stickstoffatom, an das sie gebunden sind, eine 1-Pyrrolidinyl-, 1-Piperidinyl-, 1-Azepinyl-, 4-Morpholinyl-, 4-Thiamorpholinyl-, 1-Piperazinyl-, 4-Alkyl-1-piperazinyl-, 4-Arylalkyl-1-piperazinyl-, 4-Diarylalkyl-1-piperazinylgruppe oder ein mit einem Alkyl-, Alkoxy-, Alkylthiorest, Halogenatom, einer Trifluormethyl- oder Hydroxylgruppe substituierter 1-Pyrrolidinyl-, 1-Piperidinyl- oder 1-Azepinylrest sind.
wobei das Verfahren
(i) die Behandlung eines Thioharnstoffes der Formel 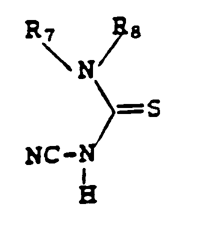 mit einem Amin der Formel 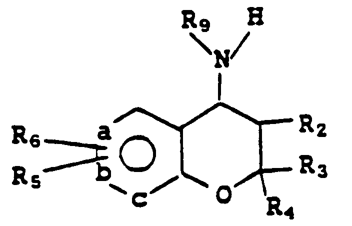 in Gegenwart eines Carbodiimids und einer Säurequelle in einem organischen Lösungsmittel oder
(ii) das Erhitzen eines Thioharnstoffes der Formel 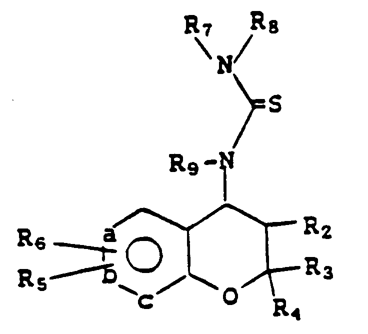 mit Mononatriumcyanamid in Gegenwart eines Carbodiimids in einem organischen Lösungsmittel oder
(iii) die Umsetzung einer Verbindung der Formel 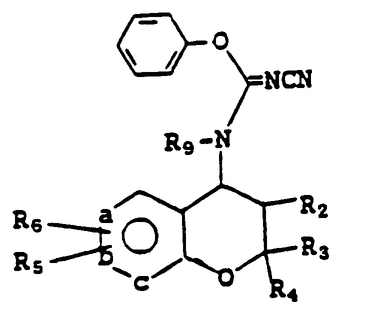 mit einem Amin der Formel
R₇R₈NH
in einem polaren Lösungsmittel umfaßt.

2. Verfahren gemäß Anspruch 1, wobei
a ein Stickstoffatom oder ein Rest der Formel =CR₅- ist;
b und c jeweils eine Gruppe der Formel =CH- sind;
R₂ eine Hydroxylgruppe oder eine Gruppe der Formel -OCOCH₃ ist;
R₃ und R₄ jeweils ein Alkylrest sind;
R₅ eine elektronenziehende Gruppe ist;
R₆ ein Wasserstoffatom, Alkylrest, ein Rest der Formel -O-Alkyl oder eine Aminogruppe ist;
R₇ ein Arylrest ist;
R₈ ein Wasserstoffatom ist;
R₉ ein Wasserstoffatom oder ein Alkylrest ist; und
n 1 oder 2 ist.

3. Verfahren gemäß Anspruch 1, wobei
a ein Stickstoffatom oder ein Rest der Formel =CR₅- ist;
b und c jeweils eine Gruppe der Formel =CH- sind;
R₂ eine trans-Hydroxylgruppe ist;
R₃ und R₄ jeweils eine Methylgruppe sind;
R₅ eine Gruppe der Formel -CN oder -NO₂ ist;
R₆ ein Wasserstoffatom ist;
R₇ eine Phenylgruppe oder ein monosubstituierter Phenylrest ist, wobei der Substituent eine Nitrogruppe, ein Halogenatom, eine Gruppe der Formel -CF₃, ein Alkylrest, eine Cyano- oder Methoxygruppe ist;
R₈ ein Wasserstoffatom ist;
R₉ ein Wasserstoffatom ist; und
n 1 ist.

4. Verfahren gemäß Anspruch 1, wobei die Produktverbindung (*trans*)-N''-Cyano-N-(6-cyano-3,4-dihydro-3-hydroxy-2,2-dimethyl-2H-1-benzopyran-4-yl)-N'-phenylguanidin ist.

5. Verfahren gemäß Anspruch 1, wobei die Produktverbindung (*trans*)-N''-Cyano-N-(3,4-dihydro-3-hydroxy-2,2-dimethyl-2H-pyrano[3,2-c]pyridin-4-yl)-phenylguanidin ist.

6. Verfahren gemäß Anspruch 1, wobei die Produktverbindung (3S-*trans*)-N''-Cyano-N-(6-cyano-3,4-dihydro-3-hydroxy-2,2-dimethyl-2H-1-benzopyran-4-yl)-N'-phenylguanidin ist.

7. Verfahren gemäß Anspruch 1, wobei die Produktverbindung (3R-*trans*)-N''-Cyano-N-(6-cyano-3,4-dihydro-3-hydroxy-2,2-dimethyl-2H-1-benzopyran-4-yl)-N'-phenylguanidin ist.

8. Verfahren gemäß Anspruch 1, wobei die Produktverbindung (*trans*)-N''-Cyano-N-(6-cyano-3,4-dihydro-3-hydroxy-2,2-dimethyl-2H-1-benzopyran-4-yl)-N'-(4-pyridinylmethyl)-guanidin ist.

9. Verfahren gemäß Anspruch 1, wobei die Produktverbindung (*trans*)-N''-Cyano-N-(6-cyano-3,4-dihydro-3-hydroxy-2,2-dimethyl-2H-1-benzopyran-4-yl)-N'-(3-pyridinylmethyl)guanidin ist.

10. Verfahren gemäß Anspruch 1, wobei die Produktverbindung (*trans*)-N''-Cyano-N-(6-ethinyl-3,4-dihydro-3-hydroxy-2,2-dimethyl-2H-1-benzopyran-4-yl)-N'-phenylguanidin ist.

11. Verfahren gemäß Anspruch 1, wobei die Produktverbindung (*trans*)-N''-Cyano-N-(3,4-dihydro-6-(phenylethinyl)-2H-1-benzopyran-4-yl)-N'-phenylguanidin ist.

12. Verfahren gemäß Anspruch 1, wobei die Produktverbindung die Struktur 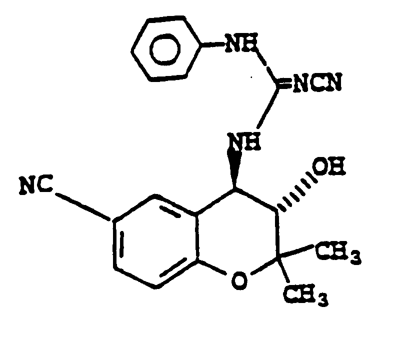 aufweist.

13. Verfahren gemäß Anspruch 1, wobei R₅ aus einem Wasserstoffatom, Alkyl-, Halogenalkyl-, Alkenyl-, Alkinyl-, Cycloalkyl-, Arylalkyl-, Cycloalkylalkylrest, einer Gruppe der Formel -CN, -NO₂, einem Rest der Formel -COR, -COOR, -CONHR, -CONR₂, einer Gruppe der Formel -CF₃, einem Rest der Formel -S-Alkyl, -SO-Alkyl, -SO₂-Alkyl, einem Halogenatom, einer Aminogruppe, einem substituierten Aminorest, einem Rest der Formel -O-Alkyl, -OCO-Alkyl, -OCONR-Alkyl, -NRCO-Alkyl, -NRCOO-Alkyl und -NRCONR₂ ausgewählt ist, wobei der Rest R in jedem der vorstehenden Reste ein Wasserstoffatom, ein Alkyl-, Aryl-, Arylalkyl-, Cycloalkyl- oder Cycloalkylalkylrest sein kann.

## Revendications (Revendications pour l'(les) Etat(s) contractant(s) suivant(s): AT, BE, CH, DE, DK, FR, GB, IT, LI, LU, NL, SE)

1. Composé de formule 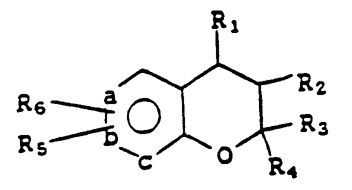 dans laquelle a, b et c sont tous =CH- ou bien l'un de a, b et c est l'azote ou =N(O)- et les autres sont =CH-;
R₁ est 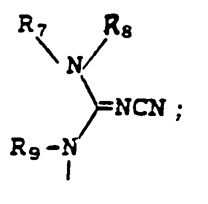 R₂ est l'hydrogène ou le groupe hydroxy ou 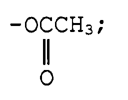 R₃ et R₄ sont tous deux, indépendamment, l'hydrogène ou un groupe alkyle ou arylalkyle, ou bien R₃ et R₄ forment, avec l'atome de carbone auquel ils sont fixés, un noyau carbocyclique de 5 à 7 chaînons;
R₅ est choisi entre l'hydrogène et les groupes alkyle, haloalkyle, alcényle, alcynyle, cycloalkyle, arylalkyle, cycloalkylalkyle, -CN, -NO₂, -COR, -COOR, -CONHR, -CONR₂, -CF₃, -S-alkyle, -SOalkyle, -SO₂alkyle, 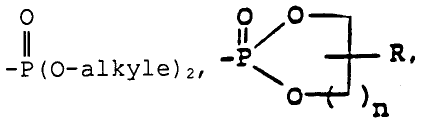 les atomes d'halogène et les groupes amine, amine substitué, O-alkyle, OCF₃, OCH₂CF₃, -OCOalkyle, -OCONRalkyle, -NRCOalkyle, NRCOOalkyle, et NRCONR₂, R pouvant être, dans chacun des groupes précédents, l'hydrogène ou un groupe alkyle, aryle, arylalkyle, cycloalkyle ou (cycloalkyl)alkyle;
R₆ est choisi entre l'hydrogène et les groupes alkyle, OH, O-alkyle, amine, amine substitué, CN et NO₂;
R₇ est choisi entre les groupes aryle, hétérocycliques et (hétérocyclo)alkyle;
R₈ est choisi entre l'hydrogène et les groupes alkyle, alcényle, aryle et arylalkyle;
R₉ est choisi entre l'hydrogène et les groupes alkyle, alcényle, aryle, arylalkyle, cycloalkyle et cycloalkylalkyle; et
n est égal à 1, 2 ou 3;
où:
"alkyle" désigne un groupe alkyle en C₁ à C₈;
"alcényle" désigne un groupe alcényle en C₂ à C₈;
"cycloalkyle" désigne un groupe cycloalkyle en C₃ à C₇;
"halogène" et "halo" désignent un atome de chlore, de brome ou de fluor;
"aryle" désigne un groupe phényle, 1-naphtyle, 2-naphtyle ou phényle, 1-naphtyle ou 2-naphtyle monosubstitué, ledit substituant étant un groupe alkyle de 1 à 4 atomes de carbone, alkylthio de 1 à 4 atomes de carbone, alcoxy de 1 à 4 atomes de carbone, un atome d'halogène ou un groupe nitro, cyano, hydroxy, amine, -NH-alkyle dans lequel le groupe alkyle a de 1 à 4 atomes de carbone, -N(alkyle)₂ dans lequel le groupe alkyle a de 1 à 4 atomes de carbone,
-CF₃, -OCHF₂, 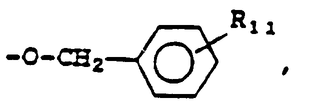 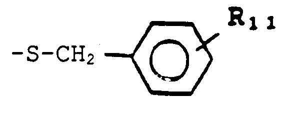 (où R₁₁ est l'hydrogène ou un groupe alkyle de 1 à 4 atomes de carbone, alcoxy de 1 à 4 atomes de carbone, alkylthio de 1 à 4 atomes de carbone, un atome d'halogène ou un groupe hydroxy ou CF₃), -O-CH₂-cycloalkyle, ou -S-CH₂-cycloalkyle ou un groupe phényle, 1-naphtyle ou 2-naphtyle bisubstitué, lesdits substituants étant choisis entre les groupes méthyle, méthoxy, méthylthio, les atomes d'halogène et les groupes CF₃, nitro, amine et OCHF₂;
"hétérocyclique" ou "hétérocyclo" désigne (a) des cycles insaturés ou complètement saturés de 5 ou 6 atomes contenant un ou deux atomes d'oxygène ou de soufre et/ou de un à quatre atomes d'azote, sous réserve que le nombre total d'hétéro-atomes du cycle soit de 4 ou moins, (b) des noyaux bicycliques dans lesquels un noyau de 5 ou 6 chaînons contenant des atomes d'oxygène, de soufre et d'azote comme défini ci-dessus est soudé à un noyau benzénique, ou (c) de tels noyaux monocycliques et bicycliques dans lesquels un atome de carbone disponible est substitué par un groupe alkyle inférieur de 1 à 4 atomes de carbone, alkylthio inférieur de 1 à 4 atomes de carbone, alcoxy inférieur de 1 à 4 atomes de carbone, un atome d'halogène ou un groupe nitro, céto, cyano, hydroxy, amine, -NH-alkyle dans lequel le groupe alkyle a de 1 à 4 atomes de carbone, -N-(alkyle)₂ dans lequel les groupes alkyle ont de 1 à 4 atomes de carbone, CF₃ ou OCHF₂, ou bien de tels noyaux monocycliques et bicycliques dans lesquels deux ou trois atomes de carbone disponibles portent des substituants choisis entre les groupes méthyle, méthoxy, méthylthio, les atomes d'halogène, et les groupes CF₃, nitro, hydroxy, amine et OCHF₂; les noyaux étant dans tous les cas reliés par un atome de carbone disponible; et
"amine substitué" désigne un groupe de formule -NZ₁Z₂ dans laquelle Z₁ est l'hydrogène ou un groupe alkyle, cycloalkyle, aryle, arylalkyle ou cycloalkylalkyle et Z₂ est un groupe alkyle, cycloalkyle, aryle, arylalkyle ou cycloalkylalkyle, ou bien Z₁ et Z₂ forment, avec l'atome d'azote auquel ils sont fixés, un groupe 1-pyrrolidinyle, 1-pipéridinyle, 1-azépinyle, 4-morpholinyle, 4-thiamorpholinyle, 1-pipérazinyle, 4-alkyl-1-pipérazinyle, 4-arylalkyl-1-pipérazinyle, 4-diarylalkyl-1-pipérazinyle, ou bien 1-pyrrolidinyle, 1-pipéridinyle ou 1-azépinyle substitué par alkyle, alcoxy, alkylthio, halogène, trifluorométhyle ou hydroxy.

2. Composé selon la revendication 1, dans lequel
a est l'azote ou =CR₅-;
b et c sont tous deux =CH-:
R₂ est un groupe hydroxy ou -OCOCH₃;
R₃ et R₄ sont tous deux des groupes alkyle;
R₅ est un groupe attracteur d'électrons;
R₆ est l'hydrogène ou un groupe alkyle, O-alkyle ou amine;
R₇ est un groupe aryle;
R₈ est l'hydrogène;
R₉ est l'hydrogène ou un groupe alkyle; et
n est égal à 1 ou 2.

3. Composé selon la revendication 1, dans lequel
a est l'azote ou =CR₅-;
b et c sont tous deux =CH-;
R₂ est un groupe trans-hydroxy;
R₃ et R₄ sont tous deux un groupe méthyle;
R₅ est -CN ou -NO₂;
R₆ est l'hydrogène;
R₇ est un groupe phényle ou phényle monosubstitué dont le substituant est un groupe nitro, un atome d'halogène ou un groupe CF₃, alkyle, cyano ou méthoxy;
R₈ est l'hydrogène;
R₉ est l'hydrogène; et
n est égal à 1.

4. La (trans)-N''-cyano-N-(6-cyano-3,4-dihydro-3-hydroxy-2,2-diméthyl-2H-1-benzopyran-4-yl)-N'-phénylguanidine.

5. La (trans)-N''-cyano-N-(3,4-dihydro-3-hydroxy-2,2-diméthyl-2H-pyrano[3,2-c]pyridin-4-yl)-phénylguanidine.

6. La (3S-trans)-N''-cyano-N-(6-cyano-3,4-dihydro-3-hydroxy-2,2-diméthyl-2H-1-benzopyran-4-yl)-N'-phénylguanidine.

7. La (3R-trans)-N''-cyano-N-(6-cyano-3,4-dihydro-3-hydroxy-2,2-diméthyl-2H-1-benzopyran-4-yl)-N'-phénylguanidine.

8. La (trans)-N''-cyano-N-(6-cyano-3,4-dihydro-3-hydroxy-2,2-diméthyl-2H-1-benzopyran-4-yl)-N'-(4-pyridinylméthyl)guanidine.

9. La (trans)-N''-cyano-N-(6-cyano-3,4-dihydro-3-hydroxy-2,2-diméthyl-2H-1-benzopyran-4-yl)-N'-(3-pyridinylméthyl)guanidine.

10. La (trans)-N''-cyano-N-(6-éthynyl-3,4-dihydro-3-hydroxy-2,2-diméthyl-2H-1-benzopyran-4-yl)-N'-phénylguanidine.

11. La (trans)-N''-cyano-N-(3,4-dihydro-6-(phényléthynyl)-2H-1-benzopyran-4-yl)-N'-phénylguanidine.

12. Composé selon la revendication 1, ayant pour formule développée

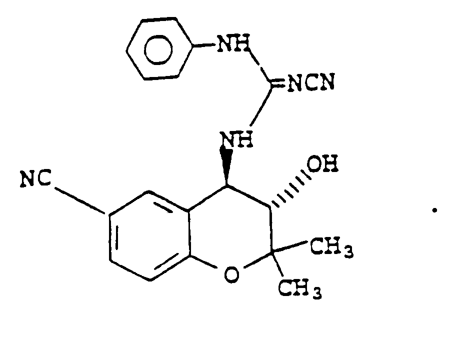

13. Utilisation d'un composé tel qu'il est défini dans l'une quelconque des revendications précédentes pour la fabrication d'un médicament permettant le traitement de l'ischémie du myocarde chez une espèce mammifère.

14. Utilisation selon la revendication 13, dans lequel ledit composé a pour formule développée

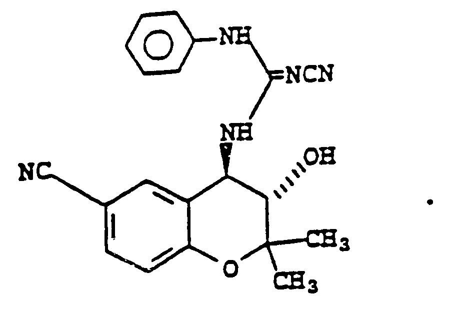

15. Utilisation d'un composé tel qu'il est défini dans l'une quelconque des revendications 1 à 12 pour la fabrication d'un médicament permettant le traitement de l'hypertension chez une espèce mammifère.

16. Composé selon la revendication 1, dans lequel
R₅ est choisi entre l'hydrogène et les groupes alkyle, haloalkyle, alcényle, alcynyle, cycloalkyle, arylalkyle, cycloalkylalkyle, -CN, -NO₂, -COR, -COOR, -CONHR, -CONR₂, -CF₃, S-alkyle, -SOalkyle, -SO₂alkyle, les atomes d'halogène et les groupes amine, amine substitué, O-alkyle, -OCOalkyle, -OCONRalkyle, -NRCOalkyle, NRCOOalkyle et NRCONR₂ où R, dans chacun des groupes précités, peut être l'hydrogène ou un groupe alkyle, aryle, arylalkyle, cycloalkyle ou cycloalkylalkyle.

17. Composé selon l'une quelconque des revendications 1 à 12 et 16, destiné à des traitements mettant en jeu l'activité d'activation des canaux potassiques.

18. Utilisation d'un composé selon l'une quelconque des revendications 1 à 12 et 16 pour la fabrication d'un médicament permettant le traitement de l'arythmie.

19. Utilisation d'un composé selon l'une quelconque des revendications 1 à 12 et 16 pour la fabrication d'un médicament permettant le traitement de l'ischémie.

20. Utilisation d'un composé selon l'une quelconque des revendications 1 à 12 et 16 pour la fabrication d'un médicament permettant le traitement de l'hypertension.

## Revendications (Revendications pour l'(les) Etat(s) contractant(s) suivant(s): ES, GR)

1. Procédé de préparation d'un composé de formule 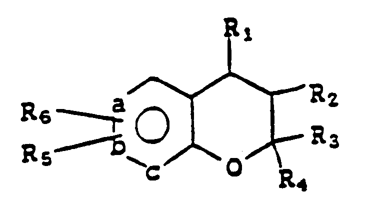 dans laquelle a, b et c sont tous =CH- ou bien l'un de a, b et c est l'azote ou =N(O)- et les autres sont =CH-;
R₁ est 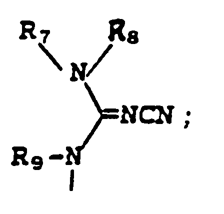 R₂ est l'hydrogène ou le groupe hydroxy ou 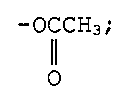 R₃ et R₄ sont tous deux, indépendamment, l'hydrogène ou un groupe alkyle ou arylalkyle, ou bien R₃ et R₄ forment, avec l'atome de carbone auquel ils sont fixés, un noyau carbocyclique de 5 à 7 chaînons;
R₅ est choisi entre l'hydrogène et les groupes alkyle, haloalkyle, alcényle, alcynyle, cycloalkyle, arylalkyle, cycloalkylalkyle, -CN, -NO₂, -COR, -COOR, -CONHR, -CONR₂, CF₃, -S-alkyle, -SOalkyle, -SO₂alkyle, 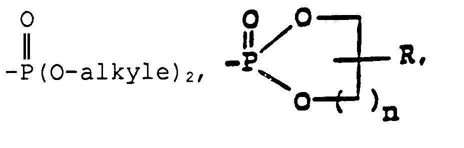 les atomes d'halogène et les groupes amine, amine substitué, O-alkyle, OCF₃, OCH₂CF₃, -OCOalkyle, -OCONRalkyle, -NRCOalkyle, NRCOOalkyle, et NRCONR₂, R pouvant être, dans chacun des groupes précédents, l'hydrogène ou un groupe alkyle, aryle, arylalkyle, cycloalkyle ou (cycloalkyl)alkyle;
R₆ est choisi entre l'hydrogène et les groupes alkyle, OH, O-alkyle, amine, amine substitué, CN et NO₂;
R₇ est choisi entre les groupes aryle, hétérocycliques et (hétérocyclo)alkyle;
R₈ est choisi entre l'hydrogène et les groupes alkyle, alcényle, aryle et arylalkyle;
R₉ est choisi entre l'hydrogène et les groupes alkyle, alcényle, aryle, arylalkyle, cycloalkyle et cycloalkylalkyle; et
n est égal à 1, 2 ou 3;
où:
"alkyle" désigne un groupe alkyle en C₁ à C₈;
"alcényle" désigne un groupe alcényle en C₂ à C₈;
"cycloalkyle" désigne un groupe cycloalkyle en C₃ à C₇;
"halogène" et "halo" désignent un atome de chlore, de brome ou de fluor;
"aryle" désigne un groupe phényle, 1-naphtyle, 2-naphtyle ou phényle, 1-naphtyle ou 2-naphtyle monosubstitué, ledit substituant étant un groupe alkyle de 1 à 4 atomes de carbone, alkylthio de 1 à 4 atomes de carbone, alcoxy de 1 à 4 atomes de carbone, un atome d'halogène ou un groupe nitro, cyano, hydroxy, amine, -NH-alkyle dans lequel le groupe alkyle a de 1 à 4 atomes de carbone, -N(alkyle)₂ dans lequel le groupe alkyle a de 1 à 4 atomes de carbone,
-CF₃, -OCHF₂, 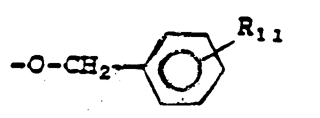 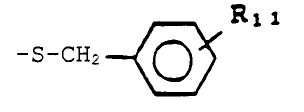 (où R₁₁ est l'hydrogène ou un groupe alkyle de 1 à 4 atomes de carbone, alcoxy de 1 à 4 atomes de carbone, alkylthio de 1 à 4 atomes de carbone, un atome d'halogène ou un groupe hydroxy ou CF₃), -O-CH₂-cycloalkyle, ou -S-CH₂-cycloalkyle ou un groupe phényle, 1-naphtyle ou 2-naphtyle bisubstitué, lesdits substituants étant choisis entre les groupes méthyle, méthoxy, méthylthio, les atomes d'halogène et les groupes CF₃, nitro, amine et OCHF₂;
"hétérocyclique" ou "hétérocyclo" désigne (a) des cycles insaturés ou complètement saturés de 5 ou 6 atomes contenant un ou deux atomes d'oxygène ou de soufre et/ou de un à quatre atomes d'azote, sous réserve que le nombre total d'hétéro-atomes du cycle soit de 4 ou moins, (b) des noyaux bicycliques dans lesquels un noyau de 5 ou 6 chaînons contenant des atomes d'oxygène, de soufre et d'azote comme défini ci-dessus est soudé à un noyau benzénique, ou (c) de tels noyaux monocycliques et bicycliques dans lesquels un atome de carbone disponible est substitué par un groupe alkyle inférieur de 1 à 4 atomes de carbone, alkylthio inférieur de 1 à 4 atomes de carbone, alcoxy inférieur de 1 à 4 atomes de carbone, un atome d'halogène ou un groupe nitro, céto, cyano, hydroxy, amine, -NH-alkyle dans lequel le groupe alkyle a de 1 à 4 atomes de carbone, -N-(alkyle)₂ dans lequel les groupes alkyle ont de 1 à 4 atomes de carbone, CF₃ ou OCHF₂, ou bien de tels noyaux monocycliques et bicycliques dans lesquels deux ou trois atomes de carbone disponibles portent des substituants choisis entre les groupes méthyle, méthoxy, méthylthio, les atomes d'halogène, et les groupes CF₃, nitro, hydroxy, amine et OCHF₂; les noyaux étant dans tous les cas reliés par un atome de carbone disponible; et
"amine substitué" désigne un groupe de formule -NZ₁Z₂ dans laquelle Z₁ est l'hydrogène ou un groupe alkyle, cycloalkyle, aryle, arylalkyle ou cycloalkylalkyle et Z₂ est un groupe alkyle, cycloalkyle, aryle, arylalkyle ou cycloalkylalkyle, ou bien Z₁ et Z₂ forment, avec l'atome d'azote auquel ils sont fixés, un groupe 1-pyrrolidinyle, 1-pipéridinyle, 1-azépinyle, 4-morpholinyle, 4-thiamorpholinyle, 1-pipérazinyle, 4-alkyl-1-pipérazinyle, 4-arylalkyl-1-pipérazinyle, 4-diarylalkyl-1-pipérazinyle, ou bien 1-pyrrolidinyle, 1-pipéridinyle ou 1-azépinyle substitué par alkyle, alcoxy, alkylthio, halogène, trifluorométhyle ou hydroxy;
procédé qui consiste
(i) à traiter une thio-urée de formule 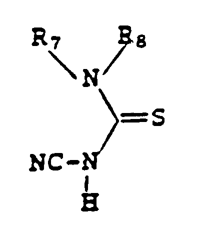 par une amine de formule 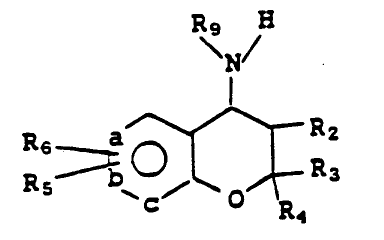 en présence d'un carbodiimide et d'une source d'acide dans un solvant organique; ou
(ii) à chauffer une thio-urée de formule 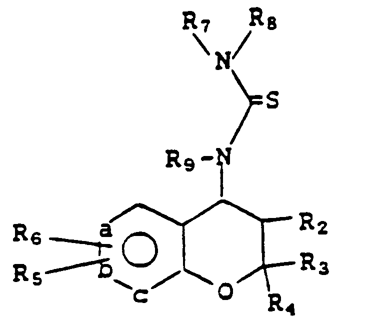 avec du cyanamide monosodique en présence d'un carbodiimide dans un solvant organique; ou
(iii) à faire réagir un composé de formule 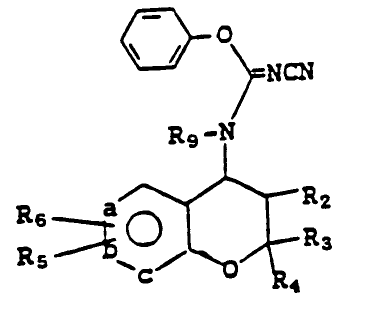 avec une amine de formule
R₇R₈NH
dans un solvant polaire.

2. Procédé selon la revendication 1, dans lequel
a est l'azote ou =CR₅-;
b et c sont tous deux =CH-:
R₂ est un groupe hydroxy ou -OCOCH₃;
R₃ et R₄ sont tous deux des groupes alkyle;
R₅ est un groupe attracteur d'électrons;
R₆ est l'hydrogène ou un groupe alkyle, O-alkyle ou amine;
R₇ est un groupe aryle;
R₈ est l'hydrogène;
R₉ est l'hydrogène ou un groupe alkyle; et
n est égal à 1 ou 2.

3. Procédé selon la revendication 1, dans lequel
a est l'azote ou =CR₅-;
b et c sont tous deux =CH-;
R₂ est un groupe trans-hydroxy;
R₃ et R₄ sont tous deux un groupe méthyle;
R₅ est -CN ou -NO₂;
R₆ est l'hydrogène;
R₇ est un groupe phényle ou phényle monosubstitué dont le substituant est un groupe nitro, un atome d'halogène ou un groupe CF₃, alkyle, cyano ou méthoxy;
R₈ est l'hydrogène;
R₉ est l'hydrogène; et
n est égal à 1.

4. Procédé selon la revendication 1, dans lequel le composé obtenu est la (trans)-N''-cyano-N-(6-cyano-3,4-dihydro-3-hydroxy-2,2-diméthyl-2H-1-benzopyran-4-yl)-N'-phénylguanidine.

5. Procédé selon la revendication 1, dans lequel le composé obtenu est la (trans)-N''-cyano-N-(3,4-dihydro-3-hydroxy-2,2-diméthyl-2H-pyrano[3,2-c]pyridin-4-yl)phénylguanidine.

6. Procédé selon la revendication 1, dans lequel le composé obtenu est la (3S-trans)-N''-cyano-N-(6-cyano-3,4-dihydro-3-hydroxy-2,2-diméthyl-2H-1-benzopyran-4-yl)-N'-phénylguanidine.

7. Procédé selon la revendication 1, dans lequel le composé obtenu est la (3R-trans)-N''-cyano-N-(6-cyano-3,4-dihydro-3-hydroxy-2,2-diméthyl-2H-1-benzopyran-4-yl)-N'-phénylguanidine.

8. Procédé selon la revendication 1, dans lequel le composé obtenu est la (trans)-N''-cyano-N-(6-cyano-3,4-dihydro-3-hydroxy-2,2-diméthyl-2H-1-benzopyran-4-yl)-N'-(4-pyridinylméthyl)guanidine.

9. Procédé selon la revendication 1, dans lequel le composé obtenu est la (trans)-N''-cyano-N-(6-cyano-3,4-dihydro-3-hydroxy-2,2-diméthyl-2H-1-benzopyran-4-yl)-N'-(3-pyridinylméthyl)guanidine.

10. Procédé selon la revendication 1, dans lequel le composé obtenu est la (trans)-N''-cyano-N-(6-éthynyl-3,4-dihydro-3-hydroxy-2,2-diméthyl-2H-1-benzopyran-4-yl)-N'-phénylguanidine.

11. Procédé selon la revendication 1, dans lequel le composé obtenu est la (trans)-N''-cyano-N-(3,4-dihydro-6-(phényléthynyl)-2H-1-benzopyran-4-yl)-N'-phénylguanidine.

12. Procédé selon la revendication 1, dans lequel le composé obtenu a pour formule développée

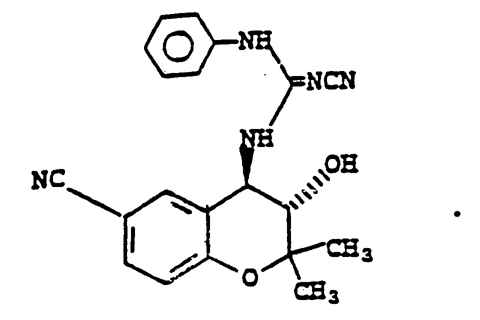

13. Procédé selon la revendication 1, dans lequel
R₅ est choisi entre l'hydrogène, les groupes alkyle, haloalkyle, alcényle, alcynyle, cycloalkyle, arylalkyle, cycloalkylalkyle, -CN, -NO₂, -COR, -COOR, -CONHR, -CONR₂, -CF₃, S-alkyle, -SOalkyle, -SO₂alkyle, les atomes d'halogène, et les groupes amine, amine substitué, O-alkyle, -OCOalkyle, -OCONRalkyle, -NRCOalkyle, NRCOOalkyle et NRCONR₂, R pouvant être, dans chacun des groupes précités, l'hydrogène ou un groupe alkyle, aryle, arylalkyle, cycloalkyle ou cycloalkylalkyle.
